(19) Europäisches Patentamt
European Patent Office
Office européen des brevets

(11) Publication number: **0 414 289 B1**

(12) **EUROPEAN PATENT SPECIFICATION**

(45) Date of publication of patent specification: **23.02.94 Bulletin 94/08**

(51) Int. Cl.$^5$: **C07D 221/20,** C07D 401/06, C07D 405/06, C07D 409/06, A61K 31/445

(21) Application number: **90202012.2**

(22) Date of filing: **23.07.90**

# (54) Spirocyclic antipsychotic agents.

(30) Priority: **26.07.89 GB 8917069**

(43) Date of publication of application: **27.02.91 Bulletin 91/09**

(45) Publication of the grant of the patent: **23.02.94 Bulletin 94/08**

(84) Designated Contracting States: **AT BE CH DE DK ES FR GB GR IT LI LU NL SE**

(56) References cited:
**US-A- 3 985 888**
**Chemical & Pharmaceutical Bulletin vol. 32, no. 9, sept. 1984, Tokio pages 3561 - 3568; Kuniko Hashigaki et al.:"Synthesis and Structure-Activity Relationship of Spiro isochromanpiperidine". Analogs for Inhibition of Histamine Release IV."**

(56) References cited:
**Archiv de Pharmazie vol. 320, no. 5, May 1987, Weinheim pages 385 - 393; Eberhard Reimann et al.: "Synthese von 3,4-Dihydro-1'-methylspiro naphtha lin-1(2H),4'-piperidinen "**
**Journal of Medicinal Chemistry vol. 21, no. 5, May 1978, Columbus Ohio pages 474 - 476; James M.Frincke et al.: "Synthesis and analgesic Activity of some Long-Acting Piperidinospiro Derivatives of Methadone"**

(73) Proprietor: **MERCK SHARP & DOHME LTD.**
**Hertford Road**
**Hoddesdon Hertfordshire EN11 9NU (GB)**

(72) Inventor: **Billington, David Charles**
**5 Limes Crescent, Hockerill**
**Bishops Stortford, Hertfordshire (GB)**
Inventor: **Chambers, Mark Stuart**
**10 Park Avenue Maisonettes, North Bushey**
**Watford, Hertfordshire (GB)**

(74) Representative: **Cole, William Gwyn et al**
**European Patent Department Merck & Co., Inc. Terlings Park Eastwick Road**
**Harlow Essex CM20 2QR (GB)**

EP 0 414 289 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a class of spirocyclic piperidine derivatives which are selective ligands at sigma recognition sites and are therefore useful as neuroleptic agents.

Various spirocyclic piperidine derivatives are already known. For example, JP-A-55-143980 generically discloses inter alia a class of spiro[1,2,3,4-tetrahydronaphthalene-1,4'-piperidine] derivatives which are substituted on the ring nitrogen atom by hydrogen or by one of a range of hydrocarbon substituents. There is, however, no specific disclosure therein of a compound possessing a spiro[1,2,3,4-tetrahydronaphthalene-1,4'-piperidine] ring system. The compounds described in JP-A-55-143980 are stated to exhibit antiallergic activity. No suggestion is made in JP-A-55-143980 that the compounds described therein may be of any assistance in solving the problem of providing an effective agent for the treatment and/or prevention of psychiatric disorders.

US-3125580 describes inter alia the preparation of a class of spiro[1,2,3,4-tetrahydronaphthalene-1,4'-piperidine] derivatives. These compounds are variously N-substituted by a 3-cyano-3,3-diphenylpropyl or 3-alkanoyl-3,3-diphenylpropyl group. The compounds described in US-3125580 are stated to be potent analgesics with a relatively long duration of activity, and to possess mydriatic activity. There is no suggestion in US-3125580 that the compounds disclosed therein may be of any use as neuroleptic agents.

US-3125580 also discloses unsubstituted, N-cyano-substituted and N-benzyl-substituted spiro[1,2,3,4-tetrahydronaphthalene-1,4'-piperidine] derivatives as intermediates. One of the intermediates described in US-3125580, namely 1'-benzylspiro[1,2,3,4-tetrahydronaphthalene-1,4'-piperidine], is stated in Chem. Pharm. Bull., 1984, 32, 3561, to have antiallergic activity via inhibition of histamine release. This document also describes the preparation of 1'-benzylspiro[1,4-dihydronaphthalene-1,4'-piperidine], but does not mention any therapeutic utility for this compound.

J. Med. Chem., 1978, 21, 474, and NIDA Research Monograph, 1987, Vol. 67 (Problems in Drug Dependency), pp. 453-489, "Evaluation of New Compounds for Opioid Activity (1985)", describe a range of spiro[1,2,3,4-tetrahydronaphthalene-1,4'-piperidine] compounds substituted on the nitrogen atom by a 3-substituted 3,3-diphenylpropyl group, which are stated to have analgesic activity.

A range of 1'-methylspiro[1,2,3,4-tetrahydronaphthalene-1,4'-piperidine] derivatives is described in Arch. Pharm. (Weinheim), 1987, 320, 385. No mention is made therein, however, of the possession of therapeutic utility by any of these compounds.

Indeed, nowhere in the prior art is there any suggestion that spiro[1,2,3,4-tetrahydronaphthalene-1,4'-piperidine] or spiro[1,4-dihydronaphthalene-1,4'-piperidine] derivatives may possess utility for the treatment and/or prevention of psychiatric disorders.

Most of the numerous currently available clinically effective antipsychotic drugs are dopamine $D_2$ receptor antagonists. As a result, they produce a characteristic spectrum of undesirable side-effects. These include endocrine effects and extrapyramidal side-effects, as well as often irreversible tardive dyskinesia. In addition, $D_2$ receptor antagonists are only palliative. They tend to alleviate only certain schizophrenic behaviour, especially the "positive" symptoms of florid delusions and hallucinations, with much less effect on the "negative" symptoms of emotional withdrawal.

From receptor binding studies, it has been shown that many effective neuroleptic agents are ligands at sigma recognition sites in the brain. Various compounds are known which are capable of interacting with the sigma recognition site, and it is considered that this interaction is significant in the manifestation of their neuroleptic properties. Most of these compounds, however, also display significant activity at the dopamine $D_2$ receptor and consequently elicit the undesirable side-effects referred to above. For example, haloperidol, a widely used neuroleptic agent, interacts equally potently with sigma sites and $D_2$ receptors.

One compound which is essentially inactive at dopamine $D_2$ receptors is rimcazole. However, whilst showing some antischizophrenic activity, rimcazole displays only moderate potency at sigma sites.

The analgesic compound N-allylnormetazocine (SKF 10047), whilst having an affinity for the sigma recognition site, also interacts strongly with the N-methyl-D-aspartate (NMDA) ion-channel complex, and thereby evokes a variety of psychotic symptoms including disorientation, excitement and hallucinations.

We have now found a class of potent, selective sigma receptor antagonists displaying negligible activity at $D_2$, NMDA and other CNS receptors, which are therefore of value as neuroleptic agents.

The present invention accordingly provides the use of a compound of formula I or a pharmaceutically acceptable salt thereof:

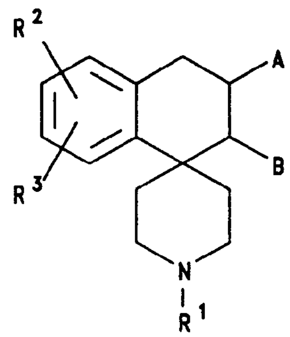

( I )

wherein

A and B each represents hydrogen, or A and B together represent a chemical bond;

$R^1$ represents hydrocarbon;

$R^2$ and $R^3$ independently represent hydrogen, hydrocarbon, halogen, cyano, trifluoromethyl, nitro, $-OR^x$, $-SR^x$, $-NR^xR^y$, $-CO_2R^x$ or $-CONR^xR^y$, or together represent methylenedioxy; and

$R^x$ and $R^y$ independently represent hydrogen or hydrocarbon;

for the manufacture of a medicament for the treatment and/or prevention of psychiatric disorders.

The term "hydrocarbon" as used herein includes straight-chained, branched and cyclic groups, including heterocyclic groups, containing up to 18 carbon atoms, suitably up to 15 carbon atoms, and conveniently up to 12 carbon atoms. Suitable hydrocarbon groups include $C_{1-6}$ alkyl, $C_{2-6}$ alkenyl, $C_{2-6}$ alkynyl, $C_{3-7}$ cycloalkyl, $C_{3-7}$ cycloalkyl($C_{1-6}$)alkyl, aryl, aryl($C_{1-6}$)alkyl, $C_{3-7}$ heterocycloalkyl, $C_{3-7}$ heterocycloalkyl($C_{1-6}$)alkyl, heteroaryl and heteroaryl($C_{1-6}$)alkyl.

Suitable alkyl groups include straight-chained and branched alkyl groups containing from 1 to 6 carbon atoms. Typical examples include methyl and ethyl groups, and straight-chained or branched propyl and butyl groups. Particular alkyl groups are n-propyl, isopropyl, n-butyl, t-butyl and n-hexyl.

Suitable alkenyl groups include straight-chained and branched alkenyl groups containing from 2 to 6 carbon atoms. Typical examples include vinyl, allyl, dimethylallyl and butenyl groups.

Suitable alkynyl groups include straight-chained and branched alkynyl groups containing from 2 to 6 carbon atoms. Typical examples include ethynyl and propargyl groups.

Suitable cycloalkyl groups include groups containing from 3 to 7 carbon atoms. Particular cycloalkyl groups are cyclopropyl and cyclohexyl.

Suitable aryl groups include phenyl and naphthyl groups.

Particular aryl($C_{1-6}$)alkyl groups include benzyl and phenethyl.

Suitable heterocycloalkyl groups include tetrahydrofuryl, pyrrolidinyl, piperidyl, piperazinyl and morpholinyl groups.

Suitable heteroaryl groups include pyridyl, quinolyl, isoquinolyl, pyridazinyl, pyrimidinyl, pyrazinyl, pyranyl, furyl, benzofuryl, thienyl, benzthienyl, imidazolyl, oxadiazolyl and thiadiazolyl groups. Particular heteroaryl groups are pyridyl, furyl and thienyl.

The hydrocarbon group may in turn be optionally substituted by one or more groups selected from $C_{1-6}$ alkyl, adamantyl, phenyl, halogen, $C_{1-6}$ haloalkyl, trifluoromethyl, hydroxy, $C_{1-6}$ alkoxy, aryloxy, $C_{1-3}$ alkylenedioxy, nitro, cyano, carboxy, $C_{2-6}$ alkoxycarbonyl, $C_{2-6}$ alkoxycarbonyl($C_{1-6}$)alkyl, $C_{2-6}$ alkylcarbonyloxy, optionally substituted arylcarbonyloxy, $C_{2-6}$ alkylcarbonyl, optionally substituted arylcarbonyl, $C_{1-6}$ alkylthio, $C_{1-6}$ alkylsulphinyl, $C_{1-6}$ alkylsulphonyl, amino, mono- or di($C_{1-6}$)alkylamino, $C_{2-6}$ alkylcarbonylamino and $C_{2-6}$ alkoxycarbonylamino.

The term "halogen" as used herein includes fluorine, chlorine, bromine and iodine, especially chlorine.

Suitable values for the substituent $R^1$ in the compounds of formula I above include optionally substituted $C_{3-6}$ alkyl, aryl($C_{1-6}$)alkyl, $C_{3-7}$ cycloalkyl-($C_{1-6}$)alkyl and heteroaryl($C_{1-6}$)alkyl. A particular group $R^1$ is benzyl.

Particularly preferred compounds of formula I above include:

1'-benzylspiro[1,2,3,4-tetrahydronaphthalene-1,4'-piperidine];

and pharmaceutically acceptable salts thereof.

In a further aspect, the invention provides a pharmaceutical composition comprising a compound of formula IA or a pharmaceutically acceptable salt thereof:

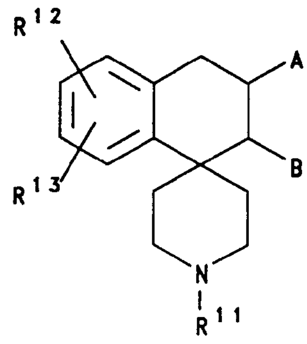

(IA)

wherein

A and B each represents hydrogen, or A and B together represent a chemical bond;

$R^{11}$ represents hydrocarbon;

$R^{12}$ and $R^{13}$ independently represent hydrogen, hydrocarbon, halogen, cyano, trifluoromethyl, nitro, $-OR^x$, $-SR^x$, $-NR^xR^y$, $-CO_2R^x$ or $-CONR^xR^y$, or together represent methylenedioxy; and

$R^x$ and $R^y$ independently represent hydrogen or hydrocarbon;

provided that when A and B both represent hydrogen and $R^{11}$ represents alkyl, allyl, aryl, aralkyl, cycloalkyl or ar(cycloalkyl), then $R^{12}$ and $R^{13}$ do not represent hydrogen, alkoxy or hydroxyalkyl;

provided also that when A, B and $R^{12}$ each represents hydrogen, and $R^{11}$ is a group of formula $-CH_2CH_2CX^1Ph_2$ in which $X^1$ represents cyano or $C_{2-7}$ alkylcarbonyl, then $R^{13}$ does not represent hydrogen or methyl in the 7-position of the tetrahydronaphthalene moiety;

further provided that when $R^{11}$ is a group of formula $-CH_2CH_2CX^2Ph_2$ in which $X^2$ represents dimethylaminocarbonyl, 1-hydroxypropyl or 1-acetoxypropyl, then A, B, $R^{12}$ and $R^{13}$ do not simultaneously represent hydrogen;

in association with one or more pharmaceutically acceptable carriers and/or excipients.

A particular pharmaceutical composition according to the invention contains 1'-benzylspiro[1,4-dihydronaphthalene-1,4'-piperidine] or a pharmaceutically acceptable salt thereof as the active ingredient.

The invention also provides a compound of formula IA as defined above or a pharmaceutically acceptable salt thereof for use in therapy.

Certain compounds falling within the definition of formula I above are novel. Accordingly, in a still further aspect the present invention provides a compound of formula II or a salt thereof:

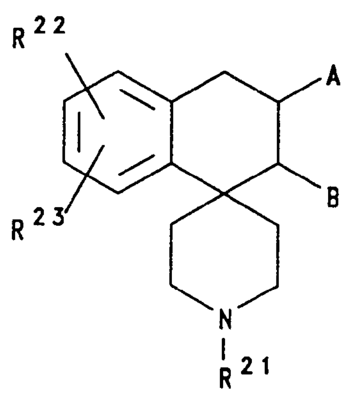

(II)

wherein

A and B each represents hydrogen, or A and B together represent a chemical bond;

$R^{21}$ represents hydrocarbon;

$R^{22}$ and $R^{23}$ independently represent hydrogen, hydrocarbon, halogen, cyano, trifluoromethyl, nitro, $-OR^x$, $-SR^x$, $-NR^xR^y$, $-CO_2R^x$ or $-CONR^xR^y$, or together represent methylenedioxy; and

$R^x$ and $R^y$ independently represent hydrogen or hydrocarbon;

provided that when A and B both represent hydrogen and $R^{21}$ represents alkyl, allyl, aryl, aralkyl, cycloalkyl or ar(cycloalkyl), then $R^{22}$ and $R^{23}$ do not represent hydrogen, alkoxy or hydroxyalkyl;

provided also that when A, B and $R^{22}$ each represents hydrogen, and $R^{21}$ is methyl, then $R^{23}$ does not represent hydroxy in the 6-position of the tetrahydronaphthalene moiety;

provided also that when A, B and $R^{22}$ each represents hydrogen, and $R^{21}$ is benzyl, then $R^{23}$ does not represent methyl in the 7-position of the tetrahydronaphthalene moiety;

provided also that when A, B and $R^{22}$ each represents hydrogen, and $R^{21}$ is a group of formula $-CH_2CH_2CX^1Ph_2$ in which $X^1$ represents cyano or $C_{2-7}$ alkylcarbonyl, then $R^{23}$ does not represent hydrogen or methyl in the 7-position of the tetrahydronaphthalene moiety;

provided also that when $R^{21}$ is a group of formula $-CH_2CH_2CX^2Ph_2$ in which $X^2$ represents dimethylaminocarbonyl, 1-hydroxypropyl or 1-acetoxypropyl, then A, B, $R^{22}$ and $R^{23}$ do not simultaneously represent hydrogen;

further provided that when A and B together represent a chemical bond and $R^{21}$ represents benzyl, then $R^{22}$ and $R^{23}$ do not simultaneously represent hydrogen.

For use in medicine, the salts of the compounds of formula II will be non-toxic pharmaceutically acceptable salts. Other salts may, however, be useful in the preparation of the compounds according to the invention or of their non-toxic pharmaceutically acceptable salts.

Suitable pharmaceutically acceptable salts of the compounds of formulae I, IA and II above include acid addition salts which may, for example, be formed by mixing a solution of the compound according to the invention with a solution of a pharmaceutically acceptable non-toxic acid such as hydrochloric acid, fumaric acid, maleic acid, succinic acid, acetic acid, citric acid, tartaric acid, carbonic acid or phosphoric acid.

Subject to the above provisos, particular values for the substituent $R^{21}$ in the compounds of formula II include optionally substituted $C_{3-6}$ alkyl, for example n-butyl and n-hexyl; optionally substituted $C_{2-6}$ alkenyl such as allyl, dimethylallyl and butenyl; optionally substituted $C_{3-7}$ cycloalkyl($C_{1-6}$)alkyl, for example cyclopropylmethyl and cyclohexylmethyl; optionally substituted aryl($C_{1-6}$)alkyl, especially benzyl, methylbenzyl, methoxybenzyl, chlorobenzyl, nitrobenzyl and phenethyl; optionally substituted $C_{3-7}$ heterocycloalkyl($C_{1-6}$)alkyl, for example tetrahydrofurylmethyl; and optionally substituted heteroaryl($C_{1-6}$)alkyl, for example furylmethyl, thienylmethyl and picolyl.

Subject to the above provisos, examples of the substituents $R^{22}$ and $R^{23}$ in the compounds of formula II above include hydrogen, halogen, $C_{1-6}$ alkyl, hydroxy and $C_{1-6}$ alkoxy. Suitably one of $R^{22}$ and $R^{23}$ represents hydrogen and the other represents hydrogen, chlorine, methyl, hydroxy or methoxy, especially hydrogen or methoxy. Preferably, $R^{22}$ and $R^{23}$ both represent hydrogen.

When $R^{22}$ and $R^{23}$ in the compounds of formula II above are other than hydrogen, they may be present at any desired position of the aromatic moiety. In a particular embodiment, these non-hydrogen groups $R^{22}$ and $R^{23}$ are present at positions 6 and 8 of the di- or tetrahydronaphthalene structure.

One sub-class of compounds according to the invention is represented by the compounds of formula IIA and salts thereof:

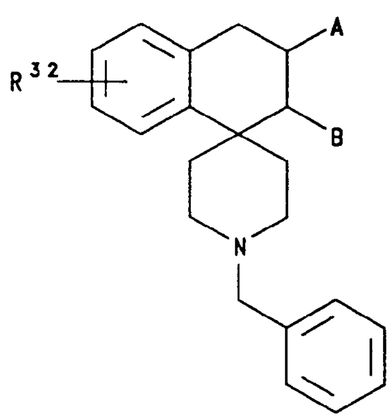

(IIA)

wherein A and B are as defined above; and $R^{32}$ represents $C_{1-6}$ alkyl, halogen, cyano, trifluoromethyl, nitro or hydroxy, especially methyl, chloro or hydroxy.

Another sub-class of compounds according to the invention is represented by the compounds of formula IIB and salts thereof:

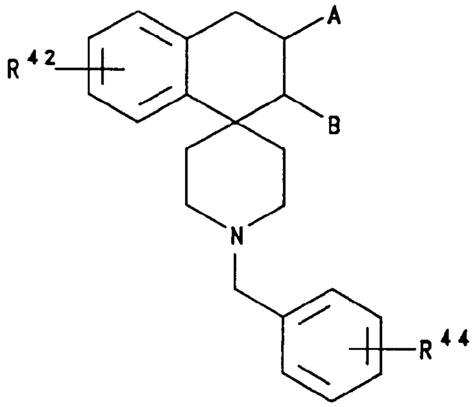

(IIB)

wherein A and B are as defined above; $R^{42}$ represents hydrogen, $C_{1-6}$ alkyl, halogen, cyano, trifluoromethyl, nitro, hydroxy or $C_{1-6}$ alkoxy; and $R^{44}$ represents $C_{1-6}$ alkyl, halogen, cyano, trifluoromethyl, nitro or $C_{1-6}$ alkoxy. Preferably, $R^{42}$ represents hydrogen, methyl, chloro, hydroxy or methoxy; and $R^{44}$ represents methyl, chloro, nitro or methoxy.

A further sub-class of compounds according to the invention is represented by the compounds of formula IIC and salts thereof:

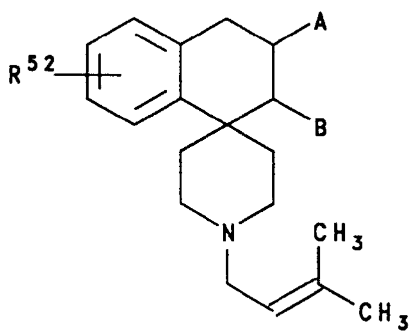

(IIC)

wherein A and B are as defined above; and $R^{52}$ represents hydrogen, $C_{1-6}$ alkyl, halogen, cyano, trifluoromethyl, nitro, hydroxy or $C_{1-6}$ alkoxy. Preferably, $R^{52}$ represents hydrogen, methyl, chloro, hydroxy or methoxy, especially hydrogen.

A still further sub-class of compounds according to the invention is represented by the compounds of formula IID and salts thereof:

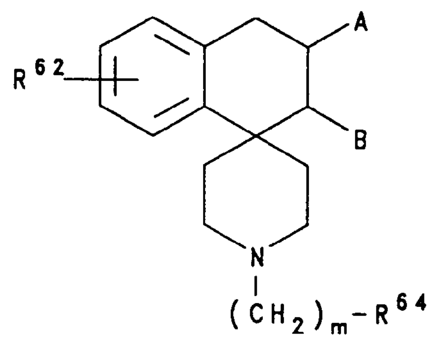

(IID)

wherein A and B are as defined above; m is 1, 2 or 3, preferably 1; $R^{64}$ is optionally substituted $C_{3-7}$ cycloalkyl; and $R^{62}$ represents hydrogen, $C_{1-6}$ alkyl, halogen, cyano, trifluoromethyl, nitro, hydroxy or $C_{1-6}$ alkoxy. Suitable values of $R^{64}$ include cyclopropyl and cyclohexyl. Preferably, $R^{62}$ represents hydrogen, methyl, chloro, hydroxy or methoxy, especially hydrogen.

A yet further sub-class of compounds according to the invention is represented by the compounds of formula IIE and salts thereof:

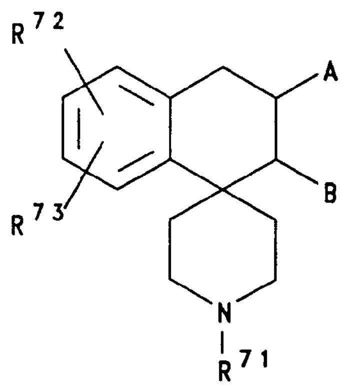

(IIE)

wherein A and B are as defined above; $R^{71}$ represents $C_{4-6}$ alkenyl, $C_{3-7}$ heterocycloalkyl($C_{1-6}$)alkyl or heteroaryl($C_{1-6}$)alkyl, any of which groups may be optionally substituted; and $R^{72}$ and $R^{73}$ independently represent hydrogen, $C_{1-6}$ alkyl, halogen, cyano, trifluoromethyl, nitro, hydroxy or $C_{1-6}$ alkoxy, especially hydrogen, methyl, chloro, hydroxy or methoxy.

Preferably, A and B each represents hydrogen in the compounds of formula IIE above. Preferred values of $R^{71}$ are butenyl, tetrahydrofurylmethyl, picolyl, furylmethyl and thienylmethyl. Preferably, $R^{72}$ and $R^{73}$ both represent hydrogen.

Specific compounds within the scope of the present invention include:
1'-(2"-picolyl)spiro[1,2,3,4-tetrahydronaphthalene-1,4'-piperidine];
1'-(4"-methoxybenzyl)spiro[1,2,3,4-tetrahydronaphthalene-1,4'-piperidine];
1'-(4"-methylbenzyl) spiro [1,2,3, 4-tetrahydronaphthalene-1,4'-piperidine];
1'-benzylspiro [1,4-dihydro-7-methoxynaphthalene-1,4'-piperidine];
1'-cyclohexylmethylspiro[1,2,3,4-tetrahydronaphthalene-1,4'-piperidine];
1'-(4"-nitrobenzyl)spiro[1,2,3,4-tetrahydronaphthalene-1,4'-piperidine];
1'-(4"-chlorobenzyl)spiro[1,2,3,4-tetrahydronaphthalene-1,4'-piperidine];
1'-(2"-tetrahydrofurylmethyl)spiro[1,2,3,4-tetrahydro-naphthalene-1,4'-piperidine];
1'-but-3"-enylspiro[1,2,3,4-tetrahydronaphthalene-1,4'-piperidine];

1'-cyclopropylmethylspiro[1,2,3,4-tetrahydronaphthalene-1,4'-piperidine];
1'-(3"-methylbut-2"-enyl)spiro[1,2,3,4-tetrahydronaphthalene-1,4'-piperidine];
1'-(2"-furylmethyl)spiro[1,2,3,4-tetrahydronaphthalene-1,4'-piperidine];
1'-(2"-thienylmethyl)spiro[1,2,3,4-tetrahydronaphthalene-1,4'-piperidine];
1'-benzylspiro[6-chloro-1,2,3,4-tetrahydronaphthalene-1,4'-piperidine];
1'-benzylspiro[6-methyl-1,2,3,4-tetrahydronaphthalene-1,4'-piperidine];
1'-benzylspiro[7-hydroxy-1,2,3,4-tetrahydronaphthalene-1,4'-piperidine];
and salts thereof.

In addition, the following compounds (each of which is referred to hereinafter as a "previously undisclosed compound of formula I") are not specifically disclosed in the prior art, and are therefore novel compounds according to the present invention:

1'-(n-butyl)spiro[1,2,3,4-tetrahydronaphthalene-1,4'-piperidine];
1'-benzylspiro[7-methoxy-1,2,3,4-tetrahydronaphthalene-1,4'-piperidine];
1'-(2"-phenylethyl)spiro[1,2,3, 4-tetrahydronaphthalene-1,4'-piperidine];
1'-n-hexylspiro[1,2,3,4-tetrahydronaphthalene-1,4'-piperidine];
1'-prop-2"-enylspiro[1,2,3,4-tetrahydronaphthalene-1,4'-piperidine];
and salts thereof.

The present invention also provides a pharmaceutical composition comprising a "previously undisclosed compound of formula I" as defined above or a pharmaceutically acceptable salt thereof in association with one or more pharmaceutically acceptable carriers and/or excipients.

The present invention further provides a "previously undisclosed compound of formula I" as defined above or a pharmaceutically acceptable salt thereof for use in therapy.

The pharmaceutical compositions of this invention are preferably in unit dosage forms such as tablets, pills, capsules, powders, granules, sterile parenteral solutions or suspensions, or suppositories, for oral, parenteral or rectal administration. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the novel compositions of the present invention may be incorporated for administration orally or by injection include aqueous solutions, suitably flavoured syrups, aqueous or oil suspensions and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone or gelatin.

In the treatment of psychiatric disorders, a suitable dosage level is about 0.01 to 250 mg/kg per day, preferably about 0.05 to 100 mg/kg per day, and especially about 0.05 to 5 mg/kg per day. The compounds may be administered on a regimen of 1 to 4 times per day.

The compounds of formula I above wherein A and B each represents hydrogen, including the novel compounds according to the invention, may be prepared by a process which comprises reducing a compound of formula III:

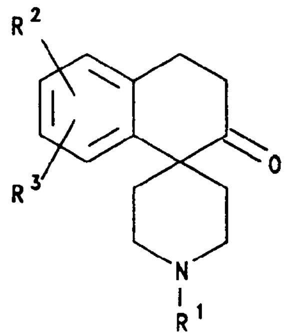

( I I I )

wherein $R^1$, $R^2$ and $R^3$ are as defined above.

The ketone of formula III may conveniently be reduced directly to the corresponding compound of formula I wherein A and B each represents hydrogen by the Wolff-Kishner method. This comprises treating the hydrazone derivative of the ketone of formula III with a strong base, e.g. potassium hydroxide, in a suitable solvent, such as diethylene glycol, at an elevated temperature, preferably the reflux temperature of the solvent.

The intermediates of formula III above may conveniently be prepared by a method analogous to that described in J. Org. Chem., 1966, 31, 3345. This comprises reacting a compound of formula $R^1-N[(CH_2)_2Z]_2$ with a β-tetralone derivative of formula IV:

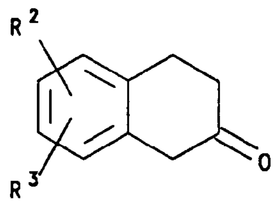

( I V )

wherein $R^1$, $R^2$ and $R^3$ are as defined above; and Z represents a leaving group, e.g. chlorine.

The β-tetralone derivatives of formula IV above, where they are not commercially available, can be prepared by the methods described in, for example, Russ. Chem. Rev. (Engl. Transl.), 1966, 35, 523; J. Org. Chem., 1968, 33, 4288; Tetrahedron Lett., 1971, 951; or J. Med. Chem., 1989, 32, 961; or by methods analogous thereto.

The compounds of formula I above wherein A and B together represent a chemical bond, including the novel compounds according to the invention, may be prepared by a process which comprises dehydrating a compound of formula V:

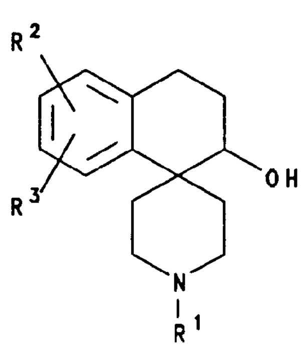

( V )

wherein $R^1$, $R^2$ and $R^3$ are as defined above.

A suitable dehydrating reagent for effecting the above conversion will, for example, be phosphorus oxychloride in the presence of pyridine.

The hydroxy intermediates of formula V above may conveniently be prepared by reduction of the keto group in the corresponding intermediate of formula III above using, for example, sodium borohydride under standard conditions.

An alternative process for preparing the compounds of formula I above, including the novel compounds according to the invention, comprises reacting a compound of formula $R^1$-L with a compound of formula VI:

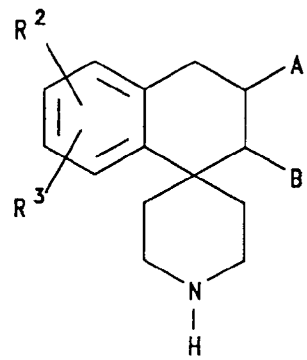

(VI)

wherein A, B, $R^1$, $R^2$ and $R^3$ are as defined above; and L represents a leaving group.

The leaving group L is suitably halogen, for example bromine.

The reaction is conveniently carried out in the presence of a mild base such as potassium carbonate, in a suitable solvent, e.g. N,N-dimethylformamide, suitably at an elevated temperature, for example a temperature in the region of 100°C.

The compounds of formula VI above wherein A and B each represents hydrogen may be prepared by a process which comprises reducing a compound of formula VII:

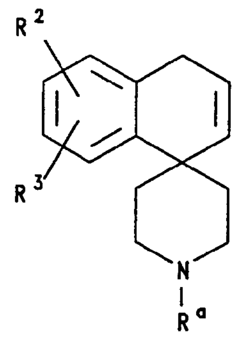

(VII)

wherein $R^2$ and $R^3$ are as defined above, and $R^a$ represents an amino-protecting group; and subsequently removing the amino-protecting group $R^a$.

An appropriate method for reducing the intermediate of formula VII is catalytic hydrogenation. A suitable catalyst is palladium hydroxide on charcoal, and the reaction is conveniently carried out in formic acid/ethanol as solvent.

Suitable examples of amino-protecting groups for the substituent $R^a$ include carboxylic acid groups such as acetyl, chloroacetyl, trifluoroacetyl, formyl, benzoyl, phthaloyl, phenylacetyl or pyridinecarbonyl; acid groups derived from carbonic acid such as ethoxycarbonyl, benzyloxycarbonyl, t-butoxycarbonyl, biphenylisopropoxycarbonyl, p-methylbenzyloxycarbonyl, p-nitrobenzyloxycarbonyl, p-bromobenzyloxycarbonyl, p-phenylazobenzyloxycarbonyl, p-(p'-methoxyphenylazo)benzyloxycarbonyl or t-amyloxycarbonyl; acid groups derived from sulphonic acid, e.g. p-toluenesulphonic acid; and other groups such as benzyl, trityl, o-nitrophenylsulphenyl or benzylidene.

Preferred amino-protecting groups are benzyl, benzyloxycarbonyl and t-butoxycarbonyl.

The removal of the amino-protecting group present in the resultant compound may be effected by an appropriate procedure depending upon the nature of the protecting group. For example, if $R^a$ represents benzyl this group may be removed in situ during hydrogenation of the intermediate of formula VII.

The intermediates of formula VII above may be prepared by appropriate variation of the method described in Chem. Pharm. Bull., 1984, 32, 3561. The synthetic route commences from a β-tetralone derivative of formula IV above and may be illustrated as follows:

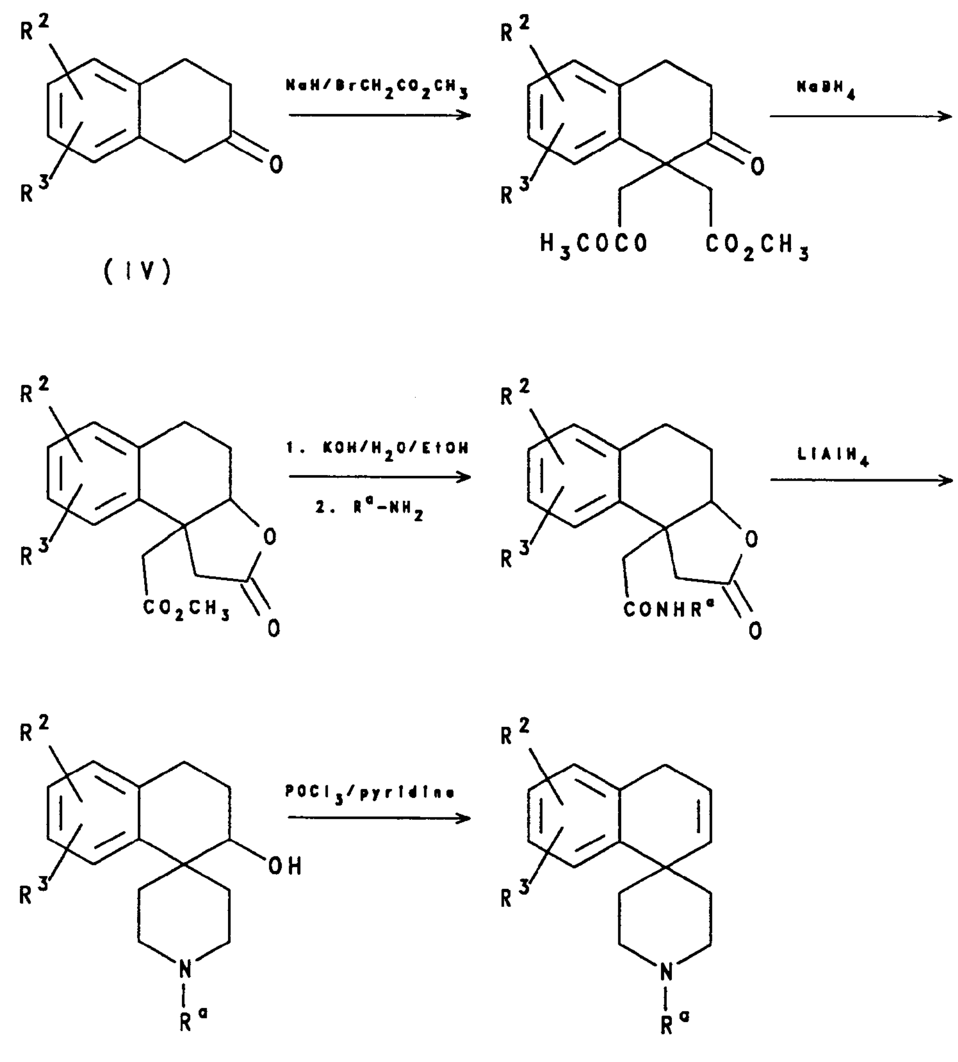


(IV)

(VII)

in which $R^2$, $R^3$ and $R^a$ are as defined above.

During any of the above synthetic sequences it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene, Protective Groups in Organic Synthesis, John Wiley & Sons, 1981. The protecting groups may be removed at a convenient subsequent stage using methods known from the art.

Displacement of Tritiated Sigma Ligand

In vitro activity

Binding of test compounds to the sigma site in vitro was determined by the method of Weber et al., Proc. Natl. Acad. Sci. USA, 1986, 83, 8784. The compounds of each of the accompanying Examples displaced tritiated di-tolyl guanidine (DTG) from sigma sites with potencies better than 200 nM.

In vivo activity

The ability of the test compounds to displace tritiated sigma ligands in vivo was determined by a modification of the method of Ferris et al., Life Sci., 1986, 38, 2329. In these experiments the sigma ligand used was tritiated (+)-SKF 10047. The compounds of accompanying Examples 2 and 3 were tested and showed an effective dose for displacing 50% of ligand ($ED_{50}$), following s.c. dosing, of better than 1.0 mg/kg in each case.

EXAMPLE 1

1'-Benzylspiro[1,4'-dihydronaphthalene-1,4'-piperidine]

Step 1: 1,1-Bis(methoxycarbonylmethyl)-2-tetralone

To a stirred solution of 2-tetralone (15g, 0.1mol) and methyl bromoacetate (47g, 0.31mol) in toluene (50ml) at 0°C, under nitrogen, was added sodium hydride (7.3g of an 80% dispersion in mineral oil, 0.24mol) portionwise. After addition of the base the reaction mixture was stirred at room temperature for 18 hours. Glacial acetic acid (18ml) was then added, followed by a few drops of concentrated hydrochloric acid and water (30ml). The mixture was then extracted with ether (3 x 200ml), and the combined ethereal layers washed with saturated sodium bicarbonate solution (2 x 70ml) and water (70ml). The organic phase was separated, dried ($MgSO_4$) and solvents removed in vacuo. The resulting orange solid was recrystallised from 1:1 petrol (60/80):ether, to give 1,1-bis(methoxycarbonylmethyl)-2-tetralone (16.9g, 57%) as white needles. m.p. 84-86°C NMR ($CDCl_3$) δ 2.90 (4H, m), 3.19 (4H, m), 3.50 (6H, s), 7.19 (4H, s). m/z (EI) 290 ($M^+$), 259, 216, 184, 157, 128, 91, 77.

Step 2: 1,2,3a,4,5,9b-Hexahydro-9b-(methoxycarbonylmethyl)naphtho[2,1-b]furan-2-one.

To a stirred solution of 1,1-bis(methoxycarbonyl-methyl)-2-tetralone (0.8g, 2.8mmol) prepared as in step 1, in ethanol (6ml), was added, portionwise, sodium borohydride (0.12g, 3.1mmol) at ambient temperature. The solution was stirred for 3 hours, then the ethanol was removed in vacuo, and the residue taken up in ethyl acetate (30ml). The organic layer was washed with water (30ml), then dried ($MgSO_4$) and the ethyl acetate removed in vacuo. The residue was chromatographed in 1:1 petrol (60/80):ether, to give 1,2,3a,4,5,9b-hexahydro-9b-(methoxycarbonylmethyl)naphtho[2,1-b]furan-2-one (0.35g, 48%) as a viscous colourless oil. NMR (60MHz, $CDCl_3$) δ 2.1 (2H, m), 2.8 (6H, m), 3.5 (3H, s), 5.0 (1H, m), 7.1 (4H, s).

Step 3: 9b-Carboxymethyl-1,2,3a,4,5,9b-hexahydronaphtho[2,1-b]furan-2-one.

A mixture of 1,2,3a,4,5,9b-hexahydro-9b-(methoxycarbonylmethyl)naphtho[2,1-b]furan-2-one (0.35g, 1.3mmol) prepared as in step 2, and 10% (w/v) potassium hydroxide in methanol:water 4:1 (4ml) was stirred at room temperature for 1.5 hours. The solvents were then removed in vacuo and the residue taken up in water (10ml). The aqueous phase was then acidified to pH1 with 1M hydrochloric acid and extracted into ethyl acetate (3 x 10ml). The combined organic layers were washed with water (10ml), dried ($MgSO_4$) and the solvent evaporated in vacuo. This gave crude 9b-carboxymethyl-1,2,3a,4,5,9b-hexahydronaphtho[2,1-b]furan-2-one (0.32g, 100%) as a colourless oil, which was used without further purification. NMR ($CDCl_3$) δ 1.98 (1H, m), 2.20 (1H, m), 2.80-3.02 (6H, m), 5.00 (1H, dd, J = 7Hz and 3Hz), 7.20 (4H, m).

Step 4: 9b-(N-Benzylcarbamoylmethyl)-1,2,3a,4,5,9b-hexahydronaphtho[2,1-b]furan-2-one

A mixture of 9b-carboxymethyl-1,2,3a,4,5,9b-hexahydronaphtho[2,1-b]furan-2-one (0.32g, 1.3mmol) prepared as in step 3, and benzylamine (140μl, 1.3mmol) were refluxed for 4 hours. After this time the solution was allowed to cool, and tetrahydrofuran (6ml) added. The resulting white solid was filtered off and recrystallised from tetrahydrofuran to give 9b-(N-benzylcarbamoylmethyl)-1,2,3a,4,5,9b-hexahydro naphtho[2,1-b]furan-2-one (0.19g, 44%) as a white solid. m.p. 197-202°C NMR ($D_6$-DMSO) δ 1.90 (1H, m), 2.19 (1H, m), 2.72 (5H, m), 3.19 (1H, d, J = 17Hz), 4.06 (1H, dd, J = 15Hz and 5Hz), 4.25 (1H, dd, J = 15Hz and 5Hz), 5.10 (1H, m), 6.92-7.40 (9H, m), 8.39 (1H, brt, J = 5Hz). m/z (EI) 335 ($M^+$), 276, 149, 91.

Step 5:1'-Benzylspiro[1,2,3,4-tetrahydro-2-hydroxynaphthalene-1,4'-piperidine]

9b-(N-Benzylcarbamoylmethyl)-1,2,3a,4,5,9b-hexahydronaphtho[2,1-b]furan-2-one (0.99g, 2.96mmol) prepared as in step 4, was added portionwise to a stirred suspension of lithium aluminium hydride (0.23g, 5.9mmol) in tetrahydrofuran (15ml), under a nitrogen atmosphere. The mixture was refluxed for 6 hours before cooling to room temperature. Water (1.0ml) was added and the mixture diluted with ethyl acetate (50ml). The organic layer was washed with water (2 x 50ml), then separated and dried ($MgSO_4$). The solvents were removed in vacuo and the residue chromatographed (eluant, ethyl acetate:ether 1:1) to give 1'-benzylspiro[1,2,3,4-tetrahydro-2-hydroxynaphthalene-1,4'-piperidine] (130mg, 14%) as a white solid.

1'-Benzylspiro[1,2,3,4-tetrahydro-2-hydroxy-naphthalene-1,4'-piperidine] (110mg, 0.36mmol) was treated with ethereal hydrogen chloride to give the title compound hydrochloride (50mg, 40%) as a white solid, after recrystallisation from 1:1 ethanol:ethyl acetate. m.p. 230-232°C NMR ($D_2O$) δ 2.00 (5H, m), 2.20 (1H, brd, J = 14Hz), 2.42 (1H, t of d, J = 10Hz and 3Hz), 2.80 (1H, brd, J = 14Hz), 3.01 (1H, m), 3.38 (3H, m), 3.58 (1H, brd, J = 10Hz), 4.38 (2H, s), 7.19-7.59 (9H, m). m/z (EI) 307 ($M^+$), 279, 216, 172, 146, 120, 91. Analysis: Requires: C, 73.34%; N, 7.62%; N, 4.07%; Found: C, 72.96%; H, 7.67%; N, 4.02%.

Step 6: 1'-Benzylspiro[1,4-dihydronaphthalene-1,4'-piperidine]

To a solution of 1'-benzylspiro[1,2,3,4-tetrahydro-2-hydroxynaphthalene-1,4'-piperidine] (30mg, 0.1mmol) prepared as in step 5, in pyridine (1.5ml) at 0°C, was added dropwise, phosphorus oxychloride (0.5mmol, 46μl). The solution was stirred at 0°C for 1 hour, then at room temperature for a further 4 hours. After this time water (0.3ml) was added dropwise, and the mixture taken up in ether (20ml). The solution was washed with water (2 x 10ml) and brine (10ml), then the organic phase was separated and dried ($MgSO_4$). The solvents were evaporated in vacuo and the residue chromatographed in 1: 1 ethyl acetate:ether to give 1'-benzylspiro[1,4-dihydronaphthalene-1,4'-piperidine] (7mg, 26%) as a colourless oil.

1'-Benzylspiro[1,4-dihydronaphthalene-1,4'-piperidine] (32mg, 0.1mmol) was treated with ethereal hydrogen chloride to give the title compound hydrochloride (19mg, 53%) as a white solid, after recrystallisation from 1:1 ethanol:ethyl acetate. m.p. 247-250°C NMR ($D_2O$) δ 1.85 (2H, brd, J = 14Hz), 2.30 (2H, t of d, J = 10Hz and 3Hz), 3.45 (6H, m), 4.41 (2H, s), 6.20 (1H, m), 6.34 (1H, d, J = 7Hz), 7.24-7.58 (9H, m). m/z (CI, $NH_3$) 290 (M+1), 91.

EXAMPLE 2

1'-Benzylspiro[1,2,3,4-tetrahydronaphthalene-1,4'-piperidine]

A solution of 1'-benzylspiro[1,4-dihydronaphthalene-1,4'-piperidine] (0.37g, 1.3mmol) prepared as in Example 1, step 6, in ethanol, (6ml) was hydrogenated at 50 p.s.i. in the presence of palladium hydroxide on carbon (Pearlman's catalyst) (50mg, 14% w/w) for 5h. The solution was filtered, and the solvent removed in vacuo. The residue was dissolved in ethanol (5ml), and benzyl bromide (0.1ml, 0.84mmol) added dropwise. The solution was stirred for 8 hours at room temperature before evaporating the ethanol. The residue was taken up in ether (10ml) and washed with 10% (w/v) aqueous potassium hydroxide (7ml). The organic layer was separated and the aqueous phase extracted further with ether (10ml). The combined ethereal layers were dried ($MgSO_4$) and the solvents removed in vacuo. The residue was chromatographed (1:1 petrol (60/80):ethyl acetate) to give 1'-benzylspiro[1,2,3,4-tetrahydronaphthalene-1,4'-piperidine] (191mg, 50%) as a colourless crystalline solid.

The hydrochloride salt was prepared using ethereal hydrogen chloride, which, after evaporation of the solvent in vacuo, gave the title compound hydrochloride (136mg, 62%) as a white solid. This was recrystallised from 1:1 ethanol:ethyl acetate to give white crystals. m.p. 245-247 (sublimed). NMR ($D_2O$) δ 1.74-1.78 (6H, m), 2.24 (2H, t of d, J = 10Hz and 4Hz), 2.80 (2H, t, J = 5Hz), 3.28 (2H, t of d, J = 10Hz and 3Hz), 3.42 (2H, m), 4.40 (2H, s), 7.20-7.60 (9H, m). m/z (CI, $NH_3$), 292 (M+1) 202, 91. Analysis: Requires: C, 76.92%; H, 7.99%; N, 4.27%; Found: C, 76.75%; H, 7.98%; N, 4.24%.

EXAMPLE 3

1'-n-Butylspiro[1,2,3,4-tetrahydronaphthalene-1,4'-piperidine]

Step 1 Spiro[1,2,3,4-tetrahydronaphthalene-1,4'-piperidine]

A solution of 1'-benzylspiro[1,4-dihydronaphthalene-1,4'-piperidine] (0.2g, 0.69mmol) prepared as in Example 1, step 6, in ethanol (10ml), containing formic acid (1ml), was hydrogenated at 50 p.s.i. in the presence of palladium hydroxide on carbon (Pearlman's catalyst) (50mg, 25% w/w) for 19 hours. The solution was filtered and the solvents removed in vacuo. The residue was taken up in ethyl acetate (50ml) and washed with 5% (w/v) potassium hydroxide in water (50ml). The organic phase was separated, and the aqueous phase extracted once more with ethyl acetate (20ml). The combined organic layers were dried ($MgSO_4$) and the solvents removed in vacuo to give spiro[1,2,3,4-tetrahydronaphthalene-1,4'-piperidine] (97mg, 70%) as a viscous oil. NMR ($D_4$-MeOH) $\delta$ 1.50 (2H, 2 x d, J = 15Hz each), 1.72 (2H, m), 1.86-2.40 (4H, m), 2.72 (2H, t, J = 7Hz), 2.90 (4H, m), 6.95-7.40 (4H, m).

The hydrochloride salt of the title compound was prepared using ethereal hydrogen chloride, which, after removal of the solvent in vacuo, gave the title compound hydrochloride (69mg, 60%) as a white solid. This was recrystallised from 1:1 ethanol:ethyl acetate. m-p. 274-278°C (sublimed). NMR ($D_2O$) $\delta$ 1.76-1.98(6H, m), 2.22 (2H, t of d, J = 10Hz and 4Hz), 2.82 (2H, t, J = 5Hz), 3.26-3.42 (4H, m), 7.20-7.54 (4H, m,). m/z (CI, $NH_3$) 202 (M+1), 128. Analysis: Requires: C, 70.72%; H, 8.48%; N, 5.89%. Found: C, 69.59%; H, 8.35%; N, 5.74%.

Step 2: 1'-n-Butylspiro[1,2,3,4-tetrahydronaphthalene-1,4'-piperidine]

To a stirred solution of spiro [1,2,3,4-tetrahydronaphthalene-1,4'-piperidine] (190mg, 0.95mmol) prepared as in step 1, in anhydrous DMF (10ml), under an atmosphere of nitrogen, was added potassium carbonate (143mg, 1.04mmol) followed by n-butyl iodide (0.11ml, 0.95mmol) dropwise. The mixture was heated at 100°C for 1 hour, after which time the solution was cooled to ambient temperature. The solvent was removed in vacuo, then ether (20ml) was added to the residue and the mixture washed with water (2 x 20ml). The organic layer was separated, dried ($MgSO_4$) and the solvent evaporated in vacuo. The crude residue was chromatographed using 95:5 dichloromethane:methanol, to give 1'-n-butylspiro[1,2,3,4-tetrahydronaphthalene-1,4'-piperidine] as a viscous oil (124mg, 51%).

The hydrochloride salt was prepared using ethereal hydrogen chloride, which, after evaporation of the solvent in vacuo, gave the title compound hydrochloride (117mg, 83%) as a white solid. mp. 245-248°C (sublimed). NMR ($D_2O$) $\delta$ 0.96 (3H, t, J = 7Hz), 1.40 (2H, m), 1.74 (4H, m), 1.93 (4H, m), 2.28 (2H, t of d, J = 12Hz and 3Hz), 2.80 (2H, t, J = 7Hz), 3.19 (4H, m), 3.52 (2H, m), 7.22-7.49 (4H, m). m/z (EI) 257 ($M^+$), 214, 129, 115, 91, 70. Analysis: Requires: C, 73.57%; H, 9.60%; N, 4.77%; Found: C, 73.23%; N, 9.45%; N, 4.70%.

EXAMPLE 4

1'-(2"-Picolyl)spiro[1,2,3,4-tetrahydronaphthalene-1,4'-piperidine]

In the same way as that described in Example 3, step 2, 1'-(2"-picolyl)spiro[1,2,3,4-tetrahydronaphthalene-1,4'-piperidine] was prepared, using spiro[1,2,3,4-tetrahydronaphthalene-1,4'-piperidine] (160mg, 0.8mmol), DMF (10ml), potassium carbonate (243mg, 1.76mmol) and picolyl chloride hydrochloride (144mg, 0.88mmol). The crude residue was chromatographed in 92:8 dichloromethane: methanol, to give 1'-(2"-picolyl)spiro[1,2,3,4-tetrahydronaphthalene-1,4'-piperidine] (68mg, 30%) as a slightly discoloured oil.

The hydrochloride salt was prepared using ethereal hydrogen chloride, to give the title compound hydrochloride (48mg, 57%) as white solid. m.p. 214-217°C NMR ($D_2O$) $\delta$ 1.75 (2H, m), 1.91 (4H, m), 2.32 (2H, m,), 2.78 (2H, t, J = 7Hz), 3.43 (4H, m), 4.58 (2H, s), 7.19 - 7.29 (3H, m), 7.46 (1H, d, J = 8Hz), 7.70 (1H, m), 7.79 (1H, d, J = 8Hz), 8.17 (1H, d of t, J = 8Hz and 1Hz), 8.72 (1H, dd, J = 6Hz and 1Hz). m/z (CI, $NH_3$) 293 (M+1), 200, 93.

EXAMPLE 5

1'-(4"-Methoxybenzyl)spiro[1,2,3,4-tetrahydronaphthalene-1,4'-piperidine]

In the same way as that described in Example 3, step 2, 1'-(4"-methoxybenzyl)spiro[1,2,3,4-tetrahydronaphthalene-1,4'-piperidine] was prepared, using spiro[1,2,3,4-tetrahydronaphthalene-1,4'-piperidine]

(158mg, 0.79mmol), DMF (10ml), potassium carbonate (119mg, 0.86mmol) and 4-methoxybenzyl chloride (0.11ml, 0.79mmol). The crude residue was chromatographed in 3:2 petrol (60/80): ethyl acetate, to give 1'-(4"-methoxybenzyl)spiro[1,2,3,4-tetrahydronaphthalene-1,4'-piperidine] (121mg, 48%) as a colourless oil.

The hydrochloride salt was prepared using ethereal hydrogen chloride, to give the title compound hydrochloride (117mg, 88%) as a white solid. m.p.258-262°C (sublimed). NMR ($D_2O$) δ 1.73-1.93 (6H, m), 2.22 (2H, t of d, J = 10Hz and 3Hz), 2.78 (2H, t, J = 6Hz), 3.27 (2H, t of d, J = 10Hz and 3Hz), 3.41 (2H, m), 3.88 (3H, s), 4.31 (2H, s), 7.09 - 7.49 (8H, m). m/z (CI, $NH_3$) 322 (M+1), 292, 200, 121, 91.

EXAMPLE 6

1'-(4"-Methylbenzyl)spiro[1,2,3,4-tetrahydronaphthalene-1,4'-piperidine]

In the same way as that described in Example 3, step 2, 1'-(4"-methylbenzyl)spiro[1,2,3,4-tetrahydronaphthalene-1,4'-piperidine] was prepared, using spiro [1,2,3,4-tetrahydronaphthalene-1,4'-piperidine] (132mg, 0.66mmol), DMF (10ml), potassium carbonate (100mg, 0.72mmol) and 4-methylbenzyl chloride (93mg, 0.66mmol). The crude residue was chromatographed in 1:1 petrol (60/80): ether, to give 1'-(4"-methylbenzyl)spiro[1,2,3,4-tetrahydronaphthalene-1,4'-piperidine] (139mg, 69%) as a colourless oil.

The hydrochloride salt was prepared using ethereal hydrogen chloride, to give the title compound hydrochloride (111mg, 71%) as a white solid. m.p.255-258°C (sublimed). NMR ($D_4$-MeOH) δ 1.64 - 1.85 (6H, m), 2.22 (5H, m), 2.67 (2H, m), 3.26 (4H, m) 4.22 (2H, s), 6.95 - 7.35 (8H, m). m/z (EI) 305 ($M^+$), 105, 77.

EXAMPLE 7

1'-Benzylspiro[1,4-dihydro-7-methoxynaphthalene-1,4'-piperidine]

Step 1: 1,1-Bis(methoxycarbonylmethyl)-7-methoxy-2-tetralone

In the same way as that described in Example 1, step 1, 1,1-bis(methoxycarbonylmethyl)-7-methoxy-2-tetralone was prepared using 7-methoxy-2-tetralone (30g, 0.17 mol), methyl bromoacetate (48ml, 0.51 mol) and sodium hydride (16g of a 60% dispersion in mineral oil, 0.41mol) in toluene (85ml). The crude residue was chromatographed in 1:1 ether: petrol (60/80) to give the title compound (32.6g, 60%) as a viscous colourless oil. NMR ($CDCl_3$) δ 2.88 (4H, m), 3.18 (4H, m), 3.50 (6H, s), 3.80 (3H, s), 6.66 (2H, m), 7.17 (1H, d, J = 9Hz).

Step 2: 1,2,3a,4,5,9b-Hexahydro-7-methoxy-9b-(methoxycarbonylmethyl)naphtho [2,1-b]furan-2-one

In the same way as that described in Example 1, step 2, the title compound was prepared using 1,1-bis(methoxycarbonylmethyl)-7-methoxy-2-tetralone (32.6g, 0.1mol) and sodium borohydride (4.2g, 0.11mol) in ethanol (170ml). The crude residue was chromatographed in 1:1 petrol (60/80): ether, to give 1,2,3a,4,5,9b-hexahydro-7-methoxy-9b-(methoxycarbonylmethyl)naphtho[2,1-b]furan-2-one (20.9g, 72%) as a viscous colourless oil. NMR ($CDCl_3$) δ 1.98 (1H, m), 2.20 (1H, m), 2.90 (6H, m), 3.60 (3H, s), 3.80 (3H, s), 4.98 (1H, dd, J = 9Hz and 5Hz), 6.70 (2H, m), 7.03 (1H, d, J = 9Hz).

Step 3: 9b-Carboxymethyl-1,2,3a,4,5,9b-hexahydro-7-methoxynaphtho[2,1-b]furan-2-one

In the same way as that described in Example 1, step 3, the title compound was prepared using 1,2,3a,4,5,9b-hexahydro-7-methoxy-9b-(methoxycarbonylmethyl)naphtho[2,1-b]furan-2-one (20.9g, 0.07mol) and potassium hydroxide in 4:1 methanol: water (182ml of 10% ($^w/v$) solution). The crude 9b-carboxymethyl-1,2,3a,4,5,9b-hexahydro-7-methoxynaphtho[2,1-b]furan-2-one (16.7g, 84%) was used without further purification. NMR ($CDCl_3$) δ 1.96 (1H, m), 2.18 (1H, m), 2.76 - 3.00 (6H, m), 3.79 (3H, s), 4.99 (1H, dd, J = 8Hz and 4Hz), 6.70 (2H, m), 7.00 (1H, d, J = 8Hz). m/z (EI) 276 ($M^+$), 260, 232, 216, 188, 171, 147, 134, 110, 91, 80.

Step 4: 9b-(N-Benzylcarbamoylmethyl)-1,2,3a,4,5,9b-hexahydro-7-methoxynaphtho[2,1-b]furan-2-one

In the same way as that described in Example 1, step 4, the title compound was prepared using 9b-carboxymethyl-1,2,3a,4,5,9b-hexyahydro-7-methoxynaphtho[2,1-b]furan-2-one (16.7g, 0.06mol) and benzylamine (6.6ml, 0.06mol). The crude 9b-(N-benzylcarbamoylmethyl)-1,2,3a,4,5,9b-hexahydro-7-methoxynaphtho[2,1-b]furan-2-one (11.1g, 51%) was isolated as a white solid, and used without any further purification. NMR ($D_6$-DMSO) δ 1.94 (1H, m), 2.19 (1H, m), 2.78 (5H, m), 3.18 (1H, d, J = 17Hz), 3.76 (3H, s), 4.06 (1H,

dd, J = 15Hz and 6Hz), 4.30 (1H, dd, J = 15Hz and 6Hz), 5.10 (1H, m), 6.68-7.42 (8H, m), 8.40 (1H, brt, J = 5Hz). m/z (EI) 365 ($M^+$), 216, 171, 149, 106, 91, 77.

Step 5: 1'-Benzylspiro[1,2,3,4-tetrahydro-2-hydroxy-7-methoxynaphthalene-1,4'-piperidine]

In the same way as that described in Example 1, step 5, the title compound was prepared using 9b-(N-benzylcarbamoylmethyl)-1,2,3a,4,5,9b-hexahydro-7-methoxynaphtho[2,1-b]furan-2-one (3.0g, 8.2mmol) and lithium aluminium hydride (0.62g, 16.4mmol) in THF (43ml). The crude residue was chromatographed in 5:1 ether:petrol (60/80) to give 1'-benzylspiro[1,2,3,4-tetrahydro-2-hydroxy-7-methoxynaphthalene-1,4'-piperidine] (150mg, 5%) as a white foam. NMR ($CDCl_3$) δ 1.64 (1H, m), 1.94-3.00 (12H, m), 3.60 (2H, s), 3.76 (3H, s), 4.40 (1H, m), 6.64 (1H, m), 6.99 (2H, m,), 7.30 (5H, m).

Step 6: 1'-Benzylspiro[1,4-dihydro-7-methoxynaphthalene-1,4'-piperidine]

In the same way as that described in Example 1, step 6, the title compound was prepared using 1'-benzylspiro[1,2,3,4-tetrahydro-2-hydroxy-7-methoxy-naphthalene-1,4'-piperidine] (150mg, 0.45mmol) and phosphorus oxychloride (0.21ml, 2.3mmol) in pyridine (4ml). The crude residue was chromatographed in 1:1 petrol (60/80):ethyl acetate to give 1'-benzylspiro[1,4-dihydro-7-methoxynaphthalene-1,4'-piperidine] (48mg, 33%) as a colourless oil. NMR ($CDCl_3$) δ 1.60 (2H, m), 2.20 (2H, t of d, J = 12Hz and 3Hz), 2.40 (2H, t of d, J = 12Hz and 2Hz), 2.84 (2H, m), 3.32 (2H, m), 3.62 (2H, s), 3.82 (3H, s), 5.99 (1H, d of t, J = 10Hz and 3Hz), 6.26 (1H, m), 6.78 (1H, dd, J = 9Hz and 2Hz), 7.04 (2H, m), 7.35 (5H, m).

The hydrochloride salt of the title compound (26mg, 0.08mmol) was prepared using ethereal hydrogen chloride, which, after removal of the solvent in vacuo, gave the title compound hydrochloride (12mg, 42%) as a white solid, after recrystallisation from 20:1 ethyl acetate:ethanol. m.p. 237-241°C (sublimed). NMR ($D_4$-MeOH) δ 1.81 (2H, m), 2.42 (2H, m), 3.36 (6H, m), 3.77 (3H, s), 4.40 (2H, s), 6.15 (1H, d of t, J = 9Hz and 1Hz), 6.33 (1H, d of t, J = 9Hz and 0.5Hz), 6.77 (1H, dd, J = 9Hz and 1Hz), 6.96 (1H, d, J = 1Hz), 7.07 (1H, d, J = 9Hz), 7.50-7.59 (5H, m), m/z (EI) 319 ($M^+$), 185, 147, 128, 91.

EXAMPLE 8

1'-Benzylspiro[1,2,3,4-tetrahydro-7-methoxynaphthalene-1,4'-piperidine]

In the same way as that described in Example 2, the title compound was prepared using 1'-benzylspiro[1,4-dihydro-7-methoxynaphthalene-1,4'-piperidine] (89mg, 0.28mmol) and palladium hydroxide on carbon (Pearlman's catalyst) (52mg, 60% ($^w$/w)) in ethanol (10ml), followed by benzyl bromide (22μl, 0.19mmol). The crude residue was chromatographed in 1:1 petrol (60/80): ethyl acetate to give 1'-benzylspiro[1,2,3,4-tetrahydro-7-methoxynaphthalene-1,4'-piperidine] (50mg, 56%) as a colourless oil.

The hydrochloride salt of the title compound was prepared using ethereal hydrogen chloride, which, after removal of the solvent in vacuo, gave the title compound hydrochloride (46mg, 83%) as a white solid. m.p. 228-232°C (sublimed). NMR ($D_4$-MeOH) δ 1.75-1.87 (4H, m), 1.95 (2H, m), 2.34 (2H, t of d, J = 12Hz and 3Hz), 2.71 (2H, t, J = 7Hz), 3.32 (4H, m), 3.75 (3H, s), 4.37 (2H, s), 6.70 (1H, dd, J = 9Hz and 1Hz), 6.92 (1H, s), 6.97 (1H, d, J = 9Hz), 7.50-7.58 (5H, m). m/z (EI) 321 ($M^+$), 147, 120, 91.

EXAMPLE 9

1'-(2"-Phenylethyl)spiro[1,2,3,4-tetrahydronaphthalene-1,4'-piperidine]

Step 1: 1'-Benzylspiro[1,2,3,4-tetrahydro-2-oxonaphthalene-1,4'-piperidine]

A stirred solution of bis (2-chloroethyl)amine hydrochloride (150g, 0.84mol), potassium carbonate (255g, 1.9mol) and benzyl bromide (110ml, 0.92mol) in DMF (1500ml) was heated at 100°C for 1 hour. The mixture was allowed to cool to ambient temperature, then the DMF removed in vacuo. The residue was taken into ethyl acetate (1000ml), then washed with water (2 x 1000ml). The organic phase was separated, dried ($MgSO_4$) and evaporated. The crude residue was chromatographed in 20:1 petrol:ether, to give N-benzylbis(2-chloroethyl)amine (36g, 18%) as a pale yellow oil.

To a stirred solution of potassium tert-butoxide (19.3g, 0.17mol) in dimethyl sulphoxide (250ml), under an atmosphere of nitrogen, was added a solution of 2-tetralone (12.6g, 0.09mol) and N-benzylbis(2-chloroethyl) amine (20g, 0.09mol) in tert-butanol (30ml), over a period of 1 hour. The solution was stirred for 18 hours at

ambient temperature then 6% hydrochloric acid was added cautiously, until pH1. The mixture was diluted with ether (600ml) and washed once more with 6% hydrochloric acid (400ml). The aqueous layers were combined, made alkaline with sodium carbonate, then extracted with ethyl acetate (5 x 200ml). The combined organic layers were dried ($MgSO_4$) and evaporated in vacuo. The crude residue was chromatographed in 1:1 petrol:ether to give 1'-benzylspiro[1,2,3,4-tetrahydro-2-oxonaphthalene-1,4'-piperidine] (4.9g, 19%) as a yellow oil. N.M.R. ($CDCl_3$) δ 2.16 (4H, m), 2.45 (2H, t of d, J = 12 and 3Hz), 2.78 (4H, m), 3.20 (2H, t, J = 7Hz), 3.56 (2H, s), 7.07-7.44 (9H, m). m/z (EI) 305 ($M^+$), 172, 147, 91.

Step 2: 1'-Benzylspiro[1,2,3,4-tetrahydronaphthalene-1,4'-piperidine]

A solution of 1'-benzylspiro[1,2,3,4-tetrahydro-2-oxonaphthalene-1,4'-piperidine] (0.73g, 2.4mmol), in potassium hydroxide (0.48g, 8.6mmol) and hydrazine hydrate (0.42ml, 8.6mmol) in diethylene glycol (6ml) was heated at reflux for 2 hours. After this time the reflux condenser was replaced by an air condenser, and heating continued for a further 2 hours. The solution was finally heated at reflux for 3 hours and then allowed to cool to ambient temperature. The mixture was diluted with ethyl acetate (100ml) and washed with water (70ml). The organic layer was separated, and the aqueous phase washed with ethyl acetate (2 x 50ml). The organic layers were combined, washed with brine (100ml), dried ($MgSO_4$) and evaporated under reduced pressure. The crude residue was chromatographed in 1:1 petrol:ether to give 1'-benzylspiro[1,2,3,4-tetrahydronaphthalene-1,4'-piperidine] as a colourless oil, which crystallised on standing. N.M.R. ($CDCl_3$) δ 1.56-1.87 (6H, m), 2.12 (2H, t of d, J = 13 and 3Hz), 2.30 (2H, m), 2.78 (4H, m), 3.59 (2H, s), 7.04-7.48 (9H, m). m/z (EI) 291 (M+), 200,146,91.

Step 3: Spiro[1,2,3,4-tetrahydronaphthalene-1,4'-piperidine]

This was prepared in the same way as that described in Example 3, Step 1, using 1'-benzylspiro[1,2,3,4-tetrahydronaphthalene-1,4'-piperidine] (0.85g, 2.9mmol), palladium hydroxide on carbon (Pearlman's catalyst) (0.22g, 25% (w/w)) and formic acid (2ml) in ethanol (20ml). The crude spiro[1,2,3,4-tetrahydronaphthalene-1,4'-piperidine] (0.37g, 64%) was used without further purification.

Spectral data is as previously described.

Step 4: 1'-(2"-Phenylethyl)spiro[1,2,3,4-tetrahydronaphthalene-1,4'-piperidine]

In the same way as that described in Example 3, using spiro[1,2,3,4-tetrahydronaphthalene-1,4'-piperidine] (300mg, 1.5mmol), DMF (10ml), potassium carbonate (270mg, 2.0mmol) and (2-bromoethyl)benzene (0.27ml, 2.0mmol). The crude residue was chromatographed in 1:1 petrol:ether, to give 1'-(2"-phenylethyl)spiro[1,2,3,4-tetrahydronaphthalene-1,4'-piperidine] (203mg, 45%) as a colourless oil.

The hydrochloride salt was prepared using ethereal hydrogen chloride, to give the title compound hydrochloride (84mg, 38%) as a white solid, after recrystallisation from ethanol/ethyl acetate. m.p. 272-274°C (sublimed). N.M.R. ($D_2O$) 1.76 (2H, m), 1.88 (4H, m), 2.31 (2H, t of d, J = 11 and 1Hz), 2.81 (2H, t, J = 6Hz), 3.16 (2H, m), 3.33 (2H, m), 3.47 (2H, m), 3.56 (2H, m), 7.22-7.55 (9H, m). m/z (CI, $NH_3$), 306 (M+1), 243, 241, 157, 128, 106, 90.

EXAMPLE 10

1'-Cyclohexylmethylspiro[1,2,3,4-tetrahydronaphthalene-1,4'-piperidine]

In the same way as that described in Example 3, using spiro[1,2,3,4-tetrahydronaphthalene-1,4'-piperidine] (176mg, 0.87mmol), DMF (10ml), potassium carbonate (145mg, 1.1mmol) and cyclohexylmethyl bromide (146μl, 1.1.mmol). The crude residue was chromatographed in 1:1 petrol:ether, to give 1'-cyclohexylmethylspiro[1,2,3,4-tetrahydronaphthalene-1,4'-piperidine] (158mg, 61%) as a colourless oil.

The hydrochloride salt was prepared using ethereal hydrogen chloride, to give the title compound hydrochloride (104mg, 59%) as a white solid, after recrystallisation from ethanol/ethyl acetate. m.p. 287-290°C (sublimed) N.M.R. ($D_2O$) δ 1.07 (2H, m), 1.28-1.98 (15H, m), 2.33 (2H, t of d, J = 11 and 1Hz), 2.80 (2H, t, J = 6Hz), 3.03 (2H, d, J = 7Hz), 3.25 (2H, m), 3.51 (2H, m), 7.22-7.30 (3H, m), 7.48 (1H, d, J = 8Hz). m/z (CI, $NH_3$) 298 (M+1).

EXAMPLE 11

1'-(4"-Nitrobenzyl)spiro[1,2,3,4-tetrahydronaphthalene-1,4'-piperidine]

In the same way as that described in Example 3, using spiro[1,2,3,4-tetrahydronaphthalene-1,4'-piperidine] (0.25g, 1.2mmol), DMF (15ml), potassium carbonate (0.2g, 1.5mmol) and 4-nitrobenzyl bromide (0.3g, 1.4mmol). The crude residue was chromatographed in 1:1 petrol:ether, to give 1'-(4"-nitrobenzyl)spiro[1,2,3,4-tetrahydronaphthalene-1,4'-piperidine] (165mg, 40%) as a pale yellow oil.

The hydrochloride salt was prepared using ethereal hydrogen chloride, to give the title compound hydrochloride (56mg, 31%) as white crystals, after recrystallisation from ethanol/ethyl acetate. m.p. 236-240°C. N.M.R. ($D_2O$) δ 1.73-1.95 (6H, m), 2.26 (2H, t of d, J = 15 and 4Hz), 2.77 (2H, t, J = 6Hz), 3.32-3.46 (4H, m), 4.50 (2H, s), 7.18-7.26 (3H, m), 7.42 (1H, d, J = 8Hz), 7.78 (1H, d, J = 9Hz), 8.35 (1H, d, J = 9Hz). m/z (EI) 336 ($M^+$), 306, 217, 191, 165, 136, 106, 78.

EXAMPLE 12

1'-(4"-Chlorobenzyl)spiro[1,2,3,4-tetrahydronaphthalene-1,4'-piperidine]

In the same way as that described in Example 3, using spiro[1,2,3,4-tetrahydronaphthalene-1,4'-piperidine] (150mg, 0.8mmol), DMF (10ml), potassium carbonate (134mg, 1mmol) and 4-chlorobenzyl chloride (144mg, 0.9mmol). The crude residue was chromatographed in 1:1 petrol:ether, to give 1-(4"-chlorobenzyl)spiro[1,2,3,4-tetrahydronaphthalene-1,4'-piperidine] (143mg, 60%) as a colourless oil.

The hydrochloride salt was prepared using ethereal hydrogen chloride, to give the title compound hydrochloride (72mg, 46%) as a white solid, after recrystallisation from ethanol/ethyl acetate. m.p. 260-264°C. N.M.R. (DMSO) δ 1.69 (4H, m), 1.85 (2H, m), 2.44 (2H, m), 2.70 (2H, t, J = 6Hz), 3.18 (4H, m), 4.34 (2H, s), 7.03-7.19 (3H, m), 7.45 (1H, d, J = 8Hz), 7.54 (2H, d, J = 8Hz), 7.67 (2H, d, J = 8Hz). m/z (EI) 325 ($M^+$), 260, 206, 180, 154, 125, 105, 91, 77.

EXAMPLE 13

1'-(2"-Tetrahydrofurfuryl)spiro[1,2,3,4-tetrahydronaphthalene-1,4'-piperidine]

In the same way as that described in Example 3, using spiro[1,2,3,4-tetrahydronaphthalene-1,4'-piperidine] (0.2g, 1.0mmol), DMF (10ml), potassium carbonate (0.17g, 1.2mmol) and tetrahydrofurfuryl bromide (0.21g, 1.3mmol). The crude residue was chromatographed in 95:5 $CH_2Cl_2$:MeOH, to give 1'-(2"-tetrahydrofurfuryl)spiro[1,2,3,4-tetrahydronaphthalene-1,4'-piperidine] (0.11g, 38%) as a pale yellow oil.

The hydrochloride salt was prepared using ethereal hydrogen chloride to give the title compound hydrochloride (45mg, 36%) as a white solid. m.p. 248-252°C. N.M.R. ($D_2O$) δ 1.65-2.03 (9H, m), 2.16 (1H, m), 2.35 (2H, m), 2.81 (2H, t, J = 6Hz), 3.25 (4H, m), 3.51 (2H, m), 3.92 (2H, m), 4.43 (1H, m), 7.21-7.33 (3H, m), 7.49 (1H, d, J = 8Hz). m/z (CI, $NH_3$) 286 (M+1), 214,85.

EXAMPLE 14

1'-n-Hexylspiro[1,2,3,4-tetrahydronaphthalene-1,4'-piperidine]

In the same way as that described in Example 3, using spiro[1,2,3,4-tetrahydronaphthalene-1,4'-piperidine] (0.2g, 1.0mmol), DMF (10ml), potassium carbonate (0.17g, 1.2mmol) and n-hexyl iodide (162μl, 1.1mmol). The crude residue was chromatographed in 1 : 1 petrol:ether, to give 1'-n-hexylspiro[1,2,3,4-tetrahydronaphthalene-1,4'-piperidine] (117mg, 39%) as a colourless oil.

The hydrochloride salt was prepared using ethereal hydrogen chloride, to give the title compound hydrochloride (90mg, 68%) as a white solid. m.p. 265-269°C. N.M.R. ($D_2O$) δ 0.89 (3H, t, J = 7Hz), 1.35 (6H, m), 1.78 (4H, m), 1.95 (4H, m), 2.28 (2H, t of d, J = 11 and 1Hz), 2.81 (2H, t, J = 6Hz), 3.22 (4H, m), 3.51 (2H, m), 7.22-7.32 (3H, m), 7.47 (1H, d, J = 8Hz). m/z (CI, $NH_3$) 286 (M+1), 214, 136, 91.

EXAMPLE 15

1'-But-3"-enylspiro[1,2,3,4-tetrahydronaphthalene-1,4'-piperidine]

In the same way as that described in Example 3, using spiro[1,2,3,4-tetrahydronaphthalene-1,4'-piperidine] (0.15g, 0.75mmol), DMF (5ml), potassium carbonate (0.13g, 0.94mmol) and 4-bromo-1-butene (0.13g, 0.98mmol). The crude residue was chromatographed in 1:1 petrol:ethyl acetate to give 1'-but-3"-enylspiro[1,2,3,4-tetrahydronaphthalene-1,4'-piperidine] (88mg, 46%) as a colourless oil.

The hydrochloride salt was prepared using ethereal hydrogen chloride, to give the title compound hydrochloride (67mg, 67%) as a white solid, after recrystallisation from ethyl acetate/ethanol. m.p. 275-279°C. N.M.R. ($D_2O$) δ 1.78 (2H, m), 1.94 (4H, m), 2.30 (2H, t of d, J = 15 and 4Hz), 2.58 (2H, m), 2.80 (2H, t, J = 6Hz), 3.29 (4H, m), 3.52 (2H, m), 5.23 (1H, d, J = 11Hz), 5.29 (1H, d, J = 17Hz), 5.85 (1H, m), 7.21-7.33 (3H, m), 7.47 (1H, d, J = 8Hz). m/z (CI, $NH_3$), 256 (M+1), 214.

EXAMPLE 16

1'-Prop-2"-enylspiro[1,2,3,4-tetrahydronaphthalene-1,4'-piperidine]

To a stirred solution of spiro [1,2,3,4-tetrahydronaphthalene-1,4'-piperidine] (0.1g, 0.5mmol) in dichloromethane (10ml), under nitrogen, was added allyl bromide (43μl, 0.5mmol) dropwise. The solution was stirred for 2 hours at room temperature, after which time it was diluted with more dichloromethane (20ml) and washed with saturated sodium bicarbonate solution (20ml). The organic layer was separated, dried ($MgSO_4$) and the solvent removed in vacuo. The crude residue was chromatographed using 90:10 dichloromethane:methanol to give the title compound (67mg, 56%) as a white solid.

The hydrochloride salt was prepared using ethereal hydrogen chloride, to give 1'-prop-2"-enylspiro[1,2,3,4-tetrahydronaphthalene-1,4'-piperidine] hydrochloride (42mg, 54%) as white needles, after recrystallisation from ethanol/ethyl acetate. m.p. 239-242°C. N.M.R. ($D_2O$) δ 1.79 (2H, m), 1.95 (4H, m), 2.28 (2H, t of d, J = 14 and 2Hz), 2.80 (2H, t, J = 6Hz), 3.26 (2H, m), 3.50 (2H, m), 3.80 (2H, d, J = 7Hz), 5.62 (2H, m), 5.99 (1H, m), 7.20-7.32 (3H, m), 7.47 (1H, d, J = 8Hz). m/z (EI) 241 ($M^+$), 214, 198, 163, 128, 115, 91, 77.

EXAMPLE 17

1'-Cyclopropylmethylspiro[1,2,3,4-tetrahydronaphthalene-1,4'-piperidine]

In the same way as that described in Example 16, using spiro[1,2,3,4-tetrahydronaphthalene-1,4'-piperidine] (0.15g, 0.75mmol), dichloromethane (10ml) and (bromomethyl) cyclopropane (0.13g, 0.97mmol). The crude residue was chromatographed (eluant 95:5 dichloromethane:methanol) to give the title compound (75mg, 40%) as a white solid.

The hydrochloride salt was prepared using ethereal hydrogen chloride, to give 1'-cyclopropylmethylspiro[1,2,3,4-tetrahydronaphthalene-1,4'-piperidine] hydrochloride (65mg, 77%) as white needles. m.p. 283-285°C. N.M.R. ($D_2O$) δ 0.44 (2H, m), 0.79 (2H, m), 1.16 (1H, m), 1.77 (2H, m), 1.94 (4H, m), 2.31 (2H, t, of d, J = 15 and 4Hz), 2.81 (2H, t, J = 6Hz), 3.09 (2H, d, J = 7Hz), 3.28 (2H, t, J = 13Hz), 3.62 (2H, m), 7.22-7.33 (3H, 16 m), 7.49 (1H, d, J = 8Hz). m/z (EI) 255 ($M^+$), 242, 214, 198, 164, 136, 110, 91, 82.

EXAMPLE 18

1'-(3"-Methylbut-2"-enyl)spiro[1,2,3,4-tetrahydronaphthalene-1,4'-piperidine]

In the same way as that described in Example 16, using spiro[1,2,3,4-tetrahydronaphthalene-1,4'-piperidine] (0.14g, 0.69mmol), dichloromethane (10ml) and 4-bromo-2-methyl-2-butene (0.1g, 0.69mmol). The crude residue was chromatographed (eluant 93:7 dichloromethane:methanol) to give the title compound (70mg, 40%) as a white solid.

The hydrochloride salt was prepared using ethereal hydrogen chloride, to give 1'-(3"-methylbut-2"-enyl)spiro[1,2,3,4-tetrahydronaphthalene-1,4'-piperidine]hydrochloride (65mg, 82%) as white needles. m.p. 274-277°C. N.M.R. ($D_2O$) δ 1.75-1.96 (12H, m), 2.25 (2H, t of d, J = 15 and 4Hz), 2.80 (2H, t, J = 6Hz), 3.23 (2H, t, J = 13Hz), 3.47 (2H, m), 3.77 (2H, d, J = 8Hz), 5.35 (1H, t, J = 8Hz), 7.21-7.32 (3H, m), 7.46 (1H, d, J = 8Hz). m/z (EI) 269 ($M^+$), 244, 214, 198, 128, 108, 91, 77.

EXAMPLE 19

1'-(2"-Furfuryl)spiro[1,2,3,4-tetrahydronaphthalene-1,4'-piperidine]

A solution of spiro[1,2,3,4-tetrahydronaphthalene-1,4'-piperidine] (0.2g, 1mmol), triethylamine (0.35ml, 2.5mmol) and 2-furoyl chloride (0.2ml, 2mmol) in dichloromethane (9ml) was stirred at room temperature, under nitrogen, for 1.5 hours. The dichloromethane was then removed in vacuo and the residue partitioned between ether (20ml) and water (20ml). The ethereal layer was separated and the aqueous layer washed once more with ether (20ml). The combined ethereal layers were dried ($MgSO_4$) and the solvent evaporated.

The residue was dissolved in anhydrous tetrahydrofuran (15ml), and to the stirred solution, under an atmosphere of nitrogen, was added, dropwise, a solution of lithium aluminium hydride in tetrahydrofuran (3ml of a 1M solution, 3mmol). After 2 hours at room temperature ethyl acetate (2ml) was added, followed by saturated ammonium chloride solution (6ml). The mixture was filtered, and the filtrate dried ($MgSO_4$) and evaporated under reduced pressure. The crude residue was chromatographed using 1:1 petrol:ether to give the title compound (103mg, 37%) as a colourless oil, which contained some 2-furfuryl alcohol.

The hydrochloride salt was prepared using ethereal hydrogen chloride, to give 1'-(2"-furfuryl)spiro[1,2,3,4-tetrahydronaphthalene-1,4'-piperidine]hydrochloride (60mg, 51%) as a white solid. m.p. 264-266°C. N.M.R. ($D_2O$) δ 1.76 (2H, m), 1.92 (4H, m), 2.26 (2H, m), 2.79 (2H, t, J = 6Hz), 3.40 (4H, m), 4.43 (2H, s), 6.57 (1H, dd, J = 3 and 2Hz), 6.75 (1H, d, J = 3Hz), 7.21-7.31 (3H, m), 7.44 (1H, d, J = 8Hz), 7.68 (1H, d, J = 2Hz). m/z (EI) 281 ($M^+$), 200, 162, 137, 110, 91, 81.

EXAMPLE 20

1'-(2"-Thienylmethyl)spiro[1,2,3,4-tetrahydronaphthalene-1,4'-piperidine]

In the same way as that described in Example 19, using spiro[1,2,3,4-tetrahydronaphthalene-1,4'-piperidine] (0.25g, 1.2mmol), 2-thiophenecarbonyl chloride (0.27ml, 2.5mmol), triethylamine (0.3g, 3.0mmol) and dichloromethane (10ml).

The resulting residue was dissolved in tetrahydrofuran (15ml) and treated with lithium aluminium hydride (3.7ml of a 1.0M solution in tetrahydrofuran, 3.7mmol). The product was chromatographed (eluant 1:1 petrol:ether) to give the title compound (153mg, 41%) as a white solid.

The hydrochloride salt was prepared using ethereal hydrogen chloride to give 1'-(2"-thiophenemethyl)spiro[1,2,3,4-tetrahydronaphthalene-1,4'-piperidine]hydrochloride (76mg, 45%) as white crystals, after recrystallisation from ethyl acetate:ethanol. m.p. 256-260°C (sublimed). N.M.R. ($D_4$-MeOH) δ 1.75-1.97 (6H, m), 2.35 (2H, t of d, J = 15 and 4Hz), 2.78 (2H, t, J = 6Hz), 3.25-3.45 (4H, m), 4.62 (2H, s), 7.14 (4H, m), 7.39 (2H, m), 7.64 (1H, d, J = 5Hz). m/z (EI) 297 ($M^+$), 248, 208, 180, 152, 129, 97, 76.

EXAMPLE 21

1'-Benzylspiro[6-Chloro-1,2,3,4-tetrahydronaphthalene-1,4'-piperidine]

Step 1: 1'-Benzylspiro[6-chloro-1,2,3,4-tetrahydro-2-oxonaphthalene-1,4'-piperidine]

To a stirred soution of potassium tert-butoxide (1.2g, 11.1mmol) in dimethyl sulphoxide (15ml), under an atmosphere of nitrogen, was added a solution of 6-chloro-2-tetralone (1.0g, 5.5mmol) and N-benzylbis(2-chloroethyl)amine (1.28g, 5.5mmol) in tert-butanol (2ml) over 15 minutes. The solution was stirred overnight at ambient temperature then 10% hydrochloric acid was added until pH1. The mixture was diluted with ether (25ml) and washed with water (50ml). The aqueous phase was separated, and the organic layer extracted once more with 10% hydrochloric acid (50ml). The aqueous layers were combined, made alkaline with sodium carbonate, then enacted with ethyl acetate (3 x 30ml). The combined organic layers were dried ($MgSO_4$) and evaporated in vacuo. The residue was chromatographed using 1:1 petrol:ether, to give 1'-benzylspiro[6-chloro-1,2,3,4-tetrahydro-2-oxonaphthalene-1,4'-piperidine] (168mg, 9%) as a yellow oil. N.M.R. ($CDCl_3$) δ 2.06 (4H, m), 2.45 (2H, t of d, J = 14 and 3Hz), 2.70 (4H, m), 3.14 (2H, t, J = 7Hz), 3.51 (2H, s), 7.10-7.40 (8H, m). m/z (EI) 339 ($M^+$), 206, 147, 128, 91.

Step 2: 1'-Benzylspiro[6-chloro-1,2,3,4-tetrahydronaphthalene-1,4'-piperidine]

In the same way as that described in Example 9, step 2, using 1'-benzylspiro[6-chloro-1,2,3,4-tetrahydro-2-oxonaphthalene-1,4'-piperidine] (1.1g, 3.3mmol), diethylene glycol (50ml), potassium hydroxide (0.86g, 15.3mmol) and hydrazine hydrate (0.6g, 1.3mmol). The crude residue was chromatographed using 1:1 petrol:ether, to give 1'-benzylspiro[6-chloro-1,2,3,4-tetrahydronaphthalene-1,4'-piperidine] (410mg, 38%) as a white solid.

The hydrochloride salt was prepared by adding ethereal hydrogen chloride to a stirred solution of the title compound (200mg, 0.6mmol) in ether (10ml). 1'-benzylspiro[6-chloro-1,2,3,4-tetrahydronaphthalene-1,4'-piperidine]hydrochloride (148mg, 67%) was isolated as white needles, after recrystallisation from ethyl acetate:ethanol. m.p. 262-268°C (sublimed). N.M.R. ($D_6$-DMSO) δ 1.67 (4H, m), 1.85 (2H, m), 2.43 (2H, m), 2.70 (2H, t, J = 6Hz), 3.18 (4H, m), 4.33 (2H, d, J = 5Hz), 7.13 (1H, s), 7.23 (1H, d, J = 9Hz), 7.47 (4H, m), 7.63 (2H, m). m/z (EI) 325 ($M^+$), 234,146,91.

EXAMPLE 22

1'-Benzylspiro[1,2,3,4-tetrahydro-6-methylnaphthalene-1,4'-piperidine]

Step 1: 1'-Benzylspiro[1,2,3,4-tetrahydro-6-methyl-2-oxonaphthalene-1,4'-piperidine]

In the same way as that described in Example 21, step 1, using 6-methyl-2-tetralone (2.4g, 15mmol), N-benzylbis(2-chloroethyl)amine (3.5g, 15mmol), potassium tert-butoxide (3.4g, 30mmol) and dimethyl sulphoxide (20ml). The crude residue was chromatographed in 1:1 petrol:ether to give 1'-benzylspiro[1,2,3,4-tetrahydro-6-methyl-2-oxonaphthalene 1,4'-piperidine] (0.84g, 17%) as a yellow oil. N.M.R. ($CDCl_3$) δ 2.08 (4H, m), 2.31 (3H, s), 2.42 (2H, t of d, J = 14 and 3Hz), 2.70 (4H, m), 3.15 (2H, t, J = 7Hz), 3.52 (2H, s), 6.93 (1H, s), 7.07 (1H, d, J = 9Hz), 7.20-7.38 (6H m).

Step 2: 1'-Benzylspiro[1,2,3,4-tetrahydro-6-methylnaphthalene-1,4'-piperidine]

In the same way as that described in Example 9, step 2, using 1'-benzylspiro[1,2,3,4-tetrahydro-6-methyl-2-oxonaphthalene-1,4'-piperidine] (84mg, 0.26mmol), diethylene glycol (3ml), potassium hydroxide (53mg, 0.95mmol) and hydrazine hydrate (46μl, 0.95mmol). The residue was chromatographed (eluant 1:1 petrol:ether) to give the title compound (28mg, 35%) as a pale yellow oil.

The hydrochloride salt was prepared using ethereal hydrogen chloride, to give 1'-benzylspiro[1,2,3,4-tetrahydro-6-methylnaphthalene-1,4'-piperidine]hydrochloride (12mg, 39%) as a white solid, after recrystallisation from ethyl acetate:ethanol. m.p. 160-164°C (sublimed). N.M.R. ($D_4$-MeOH) δ 1.73-1.91 (6H, m), 2.25 (5H, m), 2.72 (2H, t, J = 6Hz), 3.31 (4H, m), 4.35 (2H, s), 6.98 (1H, s), 7.06 (1H, d, J = 8Hz), 7.29 (1H, d, J = 8Hz), 7.54 (5H, s). m/z (EI) 305 ($M^+$), 214, 146, 115, 91.

EXAMPLE 23

1'-Benzylspiro[1,2,3,4-tetrahydro-7-hydroxynaphthalene-1,4'-piperidine]

Step 1: 1'-Benzylspiro[1,2,3,4-tetrahydro-7-methoxy-2-oxonaphthalene-1,4'-piperidine]

In the same way as that described in Example 21, step 1, using 7-methoxy-2-tetralone (10g, 0.06mol), N-benzylbis(2-chloroethyl)amine (13.9g, 0.06mol), potassium tert-butoxide (12.7g, 0.11mol), dimethyl sulphoxide (200ml) and tert-butanol (20ml). The residue was chromatographed in 1:1 petrol:ether to give 1'-benzylspiro[1,2,3,4-tetrahydro-7-methoxy-2-oxonaphthalene-1,4'-piperidine] (3.5g, 18%) as a colourless oil. N.M.R. ($CDCl_3$) δ 2.11 (4H, m), 2.44 (2H, t of d, J = 13 and 3Hz), 2.68 (2H, t, J = 7Hz), 2.76 (2H, m), 3.12 (2H, t, J = 7Hz), 3.53 (2H, s), 3.80 (3H, s), 6.74 (1H, dd, J = 8 and 3Hz), 6.98 (1H, d, J = 3Hz), 7.05 (1H, d, J = 8Hz), 7.18-7.40 (5H, m).

Step 2: 1'-Benzylspiro[1,2,3,4-tetrahydro-7-hydroxynaphthalene-1,4'-piperidine]

In the same way as that described in Example 9, step 2, using 1-benzylspiro[1,2,3,4-tetrahydro-7-methoxy-2-oxonaphthalene-1,4-piperidine] (0.66g, 1.9mmol), diethylene glycol (8ml), potassium hydroxide (0.39g, 6.9mmol) and hydrazine hydrate (0.35g, 6.9mmol). The residue was chromatographed in 1:1 petrol:ethyl acetate, to give the title compound (0.35g, 57%) as a white solid.

The hydrochloride salt was prepared by stirring 1'-benzylspiro[1,2,3,4-tetrahydro-7-hydroxynaphthalene-1,4'-piperidine] (20mg, 0.06mmol) in ethereal hydrogen chloride, to give the title compound hydrochloride (17mg, 85%) as a white crystalline solid. m.p. 165°C. N.M.R. ($D_2O$) δ 1.69-1.89 (6H, m), 2.18 (2H, t, J = 11Hz),

2.68 (2H, t, J = 6Hz), 3.29 (2H, t, J = 13Hz), 3.42 (2H, m), 4.37 (2H, s), 6.72 (1H, dd, J = 8 and 2Hz), 6.88 (1H, s), 7.05 (1H, d, J = 8Hz), 7.55 (5H, m). m/z (EI) 307 ($M^+$), 147,91.

EXAMPLE 24

Tablet Preparation

Tablets containing 1.0, 2.0, 25.0, 26.0, 50.0 and 100.0mg, respectively, of 1'-benzylspiro[1,2,3,4-tetrahydronaphthalene-1,4'-piperidine] and 1'-n-butylspiro[1,2,3,4-tetrahydronaphthalene-1,4'-piperidine] are prepared as illustrated below.

TABLE FOR DOSES CONTAINING FROM 1-25MG OF THE ACTIVE COMPOUND

| | Amount-mg. | | |
|---|---|---|---|
| Active Compound | 1.0 | 2.0 | 25.0 |
| Microcrystalline cellulose | 49.25 | 48.75 | 37.25 |
| Modified food corn starch | 49.25 | 48.75 | 37.25 |
| Magnesium stearate | 0.50 | 0.50 | 0.50 |

TABLE FOR DOSES CONTAINING FROM 26-100MG OF THE ACTIVE COMPOUND

| | Amount-mg | | |
|---|---|---|---|
| Active Compound | 26.0 | 50.0 | 100.0 |
| Microcrystalline cellulose | 52.0 | 100.0 | 200.0 |
| Modified food corn starch | 2.21 | 4.25 | 8.5 |
| Magnesium stearate | 0.39 | 0.75 | 1.5 |

All of the active compound, lactose, and a portion of the corn starch are mixed and granulated to 10% corn starch paste. The resulting granulation is sieved, dried and blended with the remainder of the corn starch and the magnesium stearate. The resulting granulation is then compressed into tablets.

**Claims**

**Claims for the following Contracting States : AT, BE, CH, DE, DK, FR, GB, IT, LI, LU, NL, SE**

**1.** The use of a compound of formula I or a pharmaceutically acceptable salt thereof:

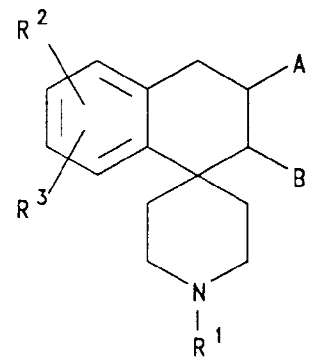

( I )

wherein

A and B each represents hydrogen, or A and B together represent a chemical bond;

$R^1$ represents hydrocarbon;

$R^2$ and $R^3$ independently represent hydrogen, hydrocarbon, halogen, cyano, trifluoromethyl, nitro, $-OR^x$, $-SR^x$, $-NR^xR^y$, $-CO_2R^x$ or $-CONR^xR^y$, or together represent methylenedioxy; and

$R^x$ and $R^y$ independently represent hydrogen or hydrocarbon; the term "hydrocarbon" as used herein includes straight-chained, branched and cyclic groups, including heterocyclic groups, containing up to 18 carbon atoms;

for the manufacture of a medicament for the treatment and/or prevention of psychiatric disorders.

**2.** The use as claimed in Claim 1 wherein the compound of formula I is selected from:
1'-benzylspiro[1,2,3,4-tetrahydronaphthalene-1,4'-piperidine];
and pharmaceutically acceptable salts thereof.

**3.** A pharmaceutical composition comprising a compound of formula IA or a pharmaceutically acceptable salt thereof:

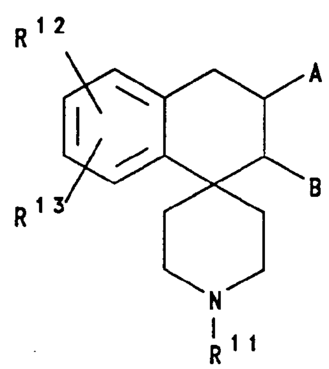

( I A )

wherein

A and B each represents hydrogen, or A and B together represent a chemical bond;

$R^{11}$ represents hydrocarbon;

$R^{12}$ and $R^{13}$ independently represent hydrogen, hydrocarbon, halogen, cyano, trifluoromethyl, nitro, $-OR^x$, $-SR^x$, $-NR^xR^y$, $-CO_2R^x$ or $-CONR^xR^y$, or together represent methylenedioxy; and

$R^x$ and $R^y$ independently represent hydrogen or hydrocarbon;

provided that when A and B both represent hydrogen and $R^{11}$ represents alkyl, allyl, aryl, aralkyl, cycloalkyl or ar(cycloalkyl), then $R^{12}$ and $R^{13}$ do not represent hydrogen, alkoxy or hydroxyalkyl;

provided also that when A, B and $R^{12}$ each represents hydrogen, and $R^{11}$ is a group of formula $-CH_2CH_2CX^1Ph_2$ in which $X^1$ represents cyano or $C_{2-7}$ alkylcarbonyl, then $R^{13}$ does not represent hydrogen

or methyl in the 7-position of the tetrahydronaphthalene moiety;

further provided that when $R^{11}$ is a group of formula $-CH_2CH_2CX^2Ph_2$ in which $X^2$ represents dimethylaminocarbonyl, 1-hydroxypropyl or 1-acetoxypropyl, then A, B, $R^{12}$ and $R^{13}$ do not simultaneously represent hydrogen; the term "hydrocarbon" as used herein includes straight-chained, branched and cyclic groups, including heterocyclic groups, containing up to 18 carbon atoms,

in association with one or more pharmaceutically acceptable carriers and/or excipients.

4. A pharmaceutical composition as claimed in Claim 3 wherein the active ingredient is 1'-benzylspiro[1,4-dihydronaphthalene-1,4'-piperidine] or a pharmaceutically acceptable salt thereof.

5. A compound of formula IA as defined in Claim 3 or a pharmaceutically acceptable salt thereof for use in therapy.

6. A compound of formula II or a salt thereof:

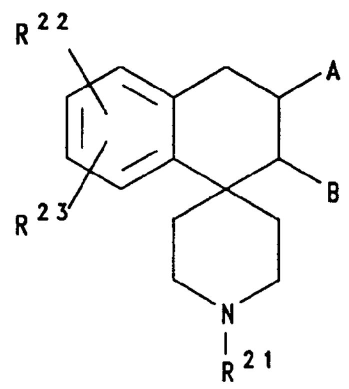

( I I )

wherein

A and B each represents hydrogen, or A and B together represent a chemical bond;

$R^{21}$ represents hydrocarbon; the term "hydrocarbon" as used herein includes straight-chained, branched and cyclic groups, including heterocyclic groups, containing up to 18 carbon atoms,

$R^{22}$ and $R^{23}$ independently represent hydrogen, hydrocarbon, halogen, cyano, trifluoromethyl, nitro, $-OR^x$, $-SR^x$, $-NR^xR^y$, $-CO_2R^x$ or $-CONR^xR^y$, or together represent methylenedioxy; and

$R^x$ and $R^y$ independently represent hydrogen or hydrocarbon;

provided that when A and B both represent hydrogen and $R^{21}$ represents alkyl, allyl, aryl, aralkyl, cycloalkyl or ar(cycloalkyl), then $R^{22}$ and $R^{23}$ do not represent hydrogen, alkoxy or hydroxyalkyl;

provided also that when A, B and $R^{22}$ each represents hydrogen, and $R^{21}$ is methyl, then $R^{23}$ does not represent hydroxy in the 6-position of the tetrahydronaphthalene moiety;

provided also that when A, B and $R^{22}$ each represents hydrogen, and $R^{21}$ is benzyl, then $R^{23}$ does not represent methyl in the 7-position of the tetrahydronaphthalene moiety;

provided also that when A, B and $R^{22}$ each represents hydrogen, and $R^{21}$ is a group of formula $-CH_2CH_2CX^1Ph_2$ in which $X^1$ represents cyano or $C_{2-7}$ alkylcarbonyl, then $R^{23}$ does not represent hydrogen or methyl in the 7-position of the tetrahydronaphthalene moiety;

provided also that when $R^{21}$ is a group of formula $-CH_2CH_2CX^2Ph_2$ in which $X^2$ represents dimethylaminocarbonyl, 1-hydroxypropyl or 1-acetoxypropyl, then A, B, $R^{22}$ and $R^{23}$ do not simultaneously represent hydrogen;

further provided that when A and B together represent a chemical bond and $R^{21}$ represents benzyl, then $R^{22}$ and $R^{23}$ do not simultaneously represent hydrogen.

7. A compound as claimed in Claim 6 represented by formula IIA and salts thereof:

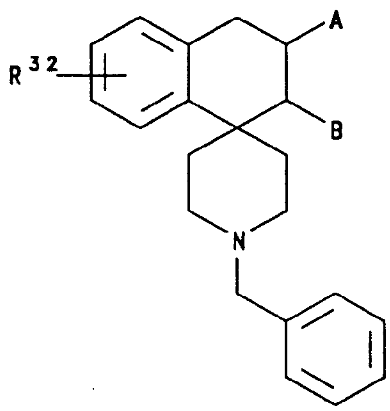

( I I A )

wherein A and B are as defined in Claim 6; and $R^{32}$ represents $C_{1-6}$ alkyl, halogen, cyano, trifluoromethyl, nitro or hydroxy.

8. A compound as claimed in Claim 6 represented by formula IIB and salts thereof:

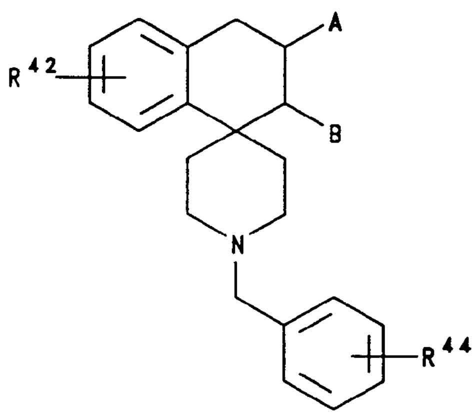

( I I B )

wherein A and B are as defined in Claim 6; $R^{42}$ represents hydrogen, $C_{1-6}$ alkyl, halogen, cyano, trifluoromethyl, nitro, hydroxy or $C_{1-6}$ alkoxy; and $R^{44}$ represents $C_{1-6}$ alkyl, halogen, cyano, trifluoromethyl, nitro or $C_{1-6}$ alkoxy.

9. A compound as claimed in Claim 6 represented by formula IIC and salts thereof:

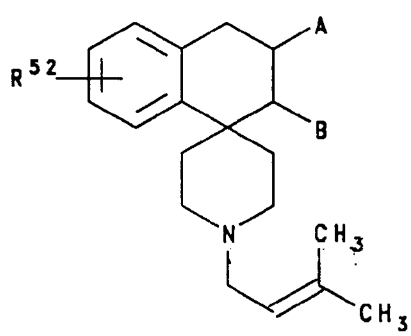

( I I C )

wherein A and B are as defined in Claim 6; and $R^{52}$ represents hydrogen, $C_{1-6}$ alkyl, halogen, cyano, tri-

fluoromethyl, nitro, hydroxy or $C_{1-6}$ alkoxy.

**10.** A compound as claimed in Claim 6 represented by formula IID and salts thereof:

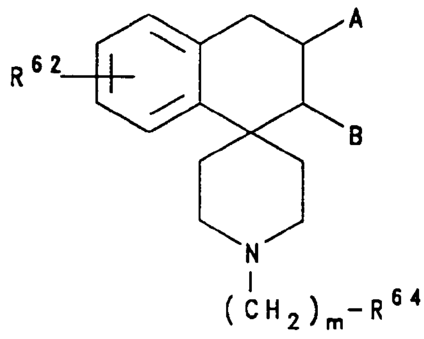

(IID)

wherein A and B are as defined in Claim 6; m is 1, 2 or 3; $R^{64}$ is optionally substituted $C_{3-7}$ cycloalkyl; and $R^{62}$ represents hydrogen, $C_{1-6}$ alkyl, halogen, cyano, trifluoromethyl, nitro, hydroxy or $C_{1-6}$ alkoxy.

**11.** A compound as claimed in Claim 6 represented by formula IIE and salts thereof:

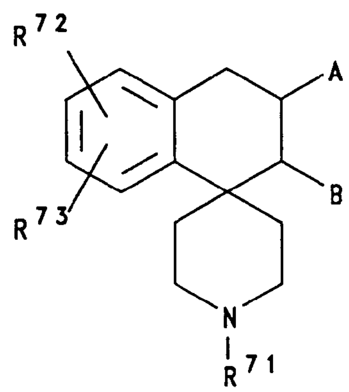

(IIE)

wherein A and B are as defined in Claim 6; $R^{71}$ represents $C_{4-6}$ alkenyl, $C_{3-7}$ heterocycloalkyl($C_{1-6}$)alkyl or heteroaryl($C_{1-6}$)alkyl, any of which groups may be optionally substituted; and $R^{72}$ and $R^{73}$ independently represent hydrogen, $C_{1-6}$ alkyl, halogen, cyano, trifluoromethyl, nitro, hydroxy or $C_{1-6}$ alkoxy.

**12.** A compound as claimed in Claim 6 selected from:
1'-(2"-picolyl)spiro[1,2,3,4-tetrahydronaphthalene-1,4'-piperidine];
1'-(4"-methoxybenzyl)spiro[1,2,3,4-tetrahydronaphthalene-1,4'-piperidine];
1'(4"-methylbenzyl)spiro[1,2,3,4-tetrahydronaphthalene-1,4'-piperidine];
1'-benzylspiro[1,4-dihydro-7-methoxynaphthalene-1,4'-piperidine];
1'-cyclohexylmethylspiro[1,2,3, 4-tetrahydronaphthalene-1,4'-piperidine];
1'-(4"-nitrobenzyl)spiro[1,2,3,4-tetrahydronaphthalene-1,4'-piperidine];
1'(4"-chlorobenzyl)spiro[1,2,3,4-tetrahydronaphthalene-1,4'-piperidine];
1'-(2"-tetrahydrofurylmethyl)spiro[1,2,3,4-tetrahydronaphthalene-1,4'-piperidine];
1'-but-3"-enylspiro[1,2,3,4-tetrahydronaphthalene-1,4-piperidine];
1'-cyclopropylmethylspiro[1,2,3,4-tetrahydronaphthalene-1,4'-piperidine];
1'-(3"-methylbut-2"-enyl)spiro[1,2,3,4-tetrahydronaphthalene-1,4,-piperidine];
1'-(2"-furylmethyl)spiro[1,2,3,4-tetrahydronaphthalene-1,4'-piperidine];

1'-(2"-thienylmethyl)spiro[1,2,3,4-tetrahydronaphthalene-1,4'-piperidine];
1'-benzylspiro[6-chloro-1,2,3,4-tetrahydronaphthalene-1,4'-piperidine];
1'-benzylspiro[6-methyl-1,2,3,4-tetrahydronaphthalene-1,4'-piperidine];
1'-benzylspiro[7-hydroxy-1,2,3,4-tetrahydronaphthalene-1,4'-piperidine];
and salts thereof.

**13.** A compound selected from:
1'-(n-butyl)spiro[1,2,3,4-tetrahydronaphthalene-1,4'-piperidine];
1'-benzylspiro[7-methoxy-1,2,3,4-tetrahydronaphthalene-1,4'-piperidine];
1'-(2"-phenylethyl)spiro[1,2,3,4-tetrahydronaphthalene-1,4'-piperidine];
1'-n-hexylspiro[1,2,3,4-tetrahydronaphthalene-1,4-piperidine];
1'-prop-2"-enylspiro[1,2,3,4-tetrahydronaphthalene-1,4'-piperidine];
and salts thereof.

**14.** A pharmaceutical composition comprising a compound as claimed in Claim 13 or a pharmaceutically acceptable salt thereof in association with one or more pharmaceutically acceptable carriers and/or excipients.

**15.** A compound as claimed in Claim 13 or a pharmaceutically acceptable salt thereof for use in therapy.

**16.** A process for the preparation of a compound of formula I as defined in Claim 1, which process comprises:
(A) for the preparation of a compound of formula I wherein A and B each represents hydrogen:
reduction of a compound of formula III:

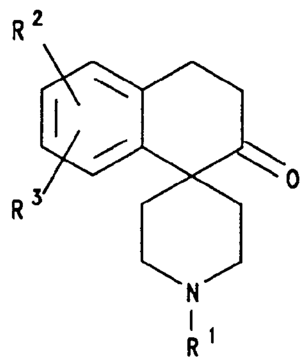

( I I I )

wherein $R^1$, $R^2$ and $R^3$ are as defined in Claim 1; or
(B) for the preparation of a compound of formula I wherein A and B together represent a chemical bond:
dehydration of a compound of formula V:

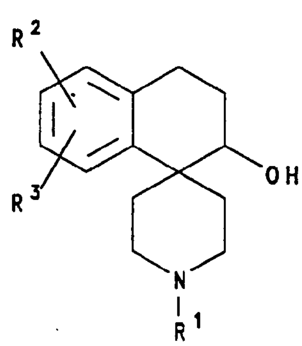

( V )

wherein $R^1$, $R^2$ and $R^3$ are as defined in Claim 1; or

(C) reacting a compound of formula $R^1$-L with a compound of formula VI:

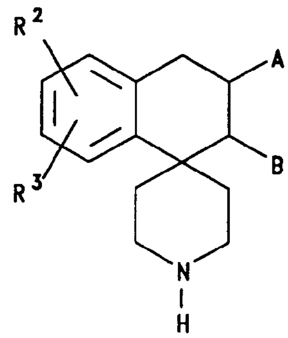

(VI)

wherein A, B, $R^1$, $R^2$ and $R^3$ are as defined in Claim 1; and L represents a leaving group.

**Claims for the following Contracting States: ES, GR**

1. A process for the preparation of a compound of formula I or a pharmaceutically acceptable salt thereof:

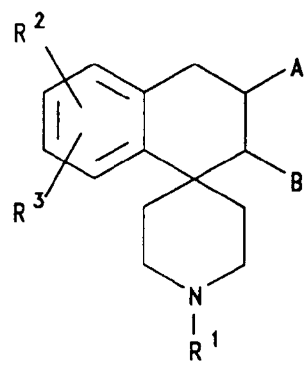

(I)

wherein

A and B each represents hydrogen, or A and B together represent a chemical bond;

$R^1$ represents hydrocarbon;

$R^2$ and $R^3$ independently represent hydrogen, hydrocarbon, halogen, cyano, trifluoromethyl, nitro, $-OR^x$, $-SR^x$, $-NR^xR^y$, $-CO_2R^x$ or $-CONR^xR^y$, or together represent methylenedioxy; and

$R^x$ and $R^y$ independently represent hydrogen or hydrocarbon; the term "hydrocarbon" as used herein includes straight-chained, branched and cyclic groups, including heterocyclic groups, containing up to 18 carbon atoms; which process comprises:

(A) for the preparation of a compound of formula I wherein A and 8 each represents hydrogen:

reduction of a compound of formula III:

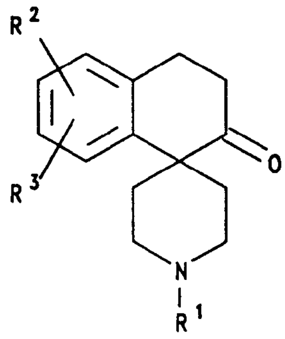

( I I I )

wherein $R^1$, $R^2$ and $R^3$ are as defined above; or

(B) for the preparation of a compound of formula I wherein A and 8 together represent a chemical bond: dehydration of a compound of formula V:

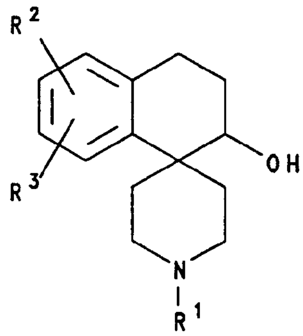

( V )

wherein $R^1$, $R^2$ and $R^3$ are as defined above; or

(C) reacting a compound of formula $R^1$-L with a compound of formula VI:

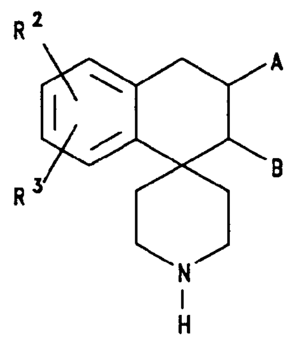

( V I )

wherein A, B, $R^1$, $R^2$ and $R^3$ are as defined above; and L represents a leaving group.

2. The use of a compound of formula I prepared as described in Claim 1, or a pharmaceutically acceptable salt thereof, for the manufacture of a medicament for the treatment and/or prevention of psychiatric disorders.

3. The use as claimed in Claim 2 wherein the compound of formula I is selected from:

1'-benzylspiro[1,2,3,4-tetrahydronaphthalene-1,4-piperidine];

and pharmaceutically acceptable salts thereof.

4. A process for the preparation of a pharmaceutical composition which comprises mixing a compound of formula IA or a pharmaceutically acceptable salt thereof:

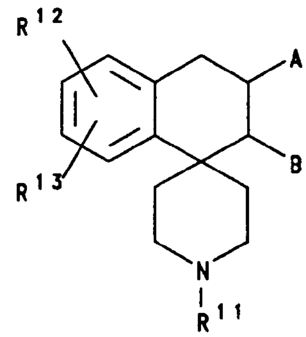

( I A )

wherein

A and B each represents hydrogen, or A and B together represent a chemical bond;

$R^{11}$ represents hydrocarbon;

$R^{12}$ and $R^{13}$ independently represent hydrogen, hydrocarbon, halogen, cyano, trifluoromethyl, nitro, $-OR^x$, $-SR^x$, $-NR^xR^y$, $-CO_2R^x$ or $-CONR^xR^y$, or together represent methylenedioxy; and

$R^x$ and $R^y$ independently represent hydrogen or hydrocarbon;

provided that when A and B both represent hydrogen and $R^{11}$ represents alkyl, allyl, aryl, aralkyl, cycloalkyl or ar(cycloalkyl), then $R^{12}$ and $R^{13}$ do not represent hydrogen, alkoxy or hydroxyalkyl;

provided also that when A, B and $R^{12}$ each represents hydrogen, and $R^{11}$ is a group of formula $-CH_2CH_2CX^1Ph_2$ in which $X^1$ represents cyano or $C_{2-7}$ alkylcarbonyl, then $R^{13}$ does not represent hydrogen or methyl in the 7-position of the tetrahydronaphthalene moiety;

further provided that when $R^{11}$ is a group of formula $-CH_2CH_2CX^2Ph_2$ in which $X^2$ represents dimethylaminocarbonyl, 1-hydroxypropyl or 1-acetoxypropyl, then A, B, $R^{12}$ and $R^{13}$ do not simultaneously represent hydrogen; the term "hydrocarbon" as used herein includes straight-chained, branched and cyclic groups, including heterocyclic groups, containing up to 18 carbon atoms;

with one or more pharmaceutically acceptable carriers and/or excipients.

5. A process as claimed in Claim 4 for the preparation of a pharmaceutical composition wherein the active ingredient is 1'-benzylspiro[1,4-dihydronaphthalene-1,4'-piperidine] or a pharmaceutically acceptable salt thereof.

6. A process as claimed in Claim 1 for the preparation of a compound of formula II or a salt thereof:

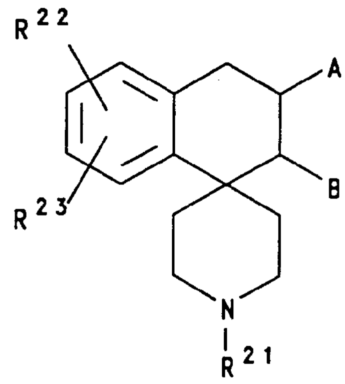

( I I )

wherein

A and B each represents hydrogen, or A and B together represent a chemical bond;

$R^{21}$ represents hydrocarbon; the term "hydrocarbon" as used herein includes straight-chained, branched and cyclic groups, including heterocyclic groups, containing up to 18 carbon atoms;

$R^{22}$ and $R^{23}$ independently represent hydrogen, hydrocarbon, halogen, cyano, trifluoromethyl, nitro, $-OR^x$, $-SR^x$, $-NR^xR^y$, $-CO_2R^x$ or $-CONR^xR^y$, or together represent methylenedioxy; and

$R^x$ and $R^y$ independently represent hydrogen or hydrocarbon;

provided that when A and B both represent hydrogen and $R^{21}$ represents alkyl, allyl, aryl, aralkyl, cycloalkyl or ar(cycloalkyl), then $R^{22}$ and $R^{23}$ do not represent hydrogen, alkoxy or hydroxyalkyl;

provided also that when A, B and $R^{22}$ each represents hydrogen, and $R^{21}$ is methyl, then $R^{23}$ does not represent hydroxy in the 6-position of the tetrahydronaphthalene moiety;

provided also that when A, B and $R^{22}$ each represents hydrogen, and $R^{21}$ is benzyl, then $R^{23}$ does not represent methyl in the 7-position of the tetrahydronaphthalene moiety;

provided also that when A, B and $R^{22}$ each represents hydrogen, and $R^{21}$, is a group of formula $-CH_2CH_2CX^1Ph_2$ in which $X^1$ represents cyano or $C_{2-7}$ alkylcarbonyl, then $R^{23}$ does not represent hydrogen or methyl in the 7-position of the tetrahydronaphthalene moiety;

provided also that when $R^{21}$ is a group of formula $-CH_2CH_2CX^2Ph_2$ in which $X^2$ represents dimethylaminocarbonyl, 1-hydroxypropyl or 1-acetoxypropyl, then A, B, $R^{22}$ and $R^{23}$ do not simultaneously represent hydrogen;

further provided that when A and B together represent a chemical bond and $R^{21}$ represents benzyl, then $R^{22}$ and $R^{23}$ do not simultaneously represent hydrogen.

7. A process as claimed in Claim 6 for the preparation of a compound represented by formula IIA and salts thereof:

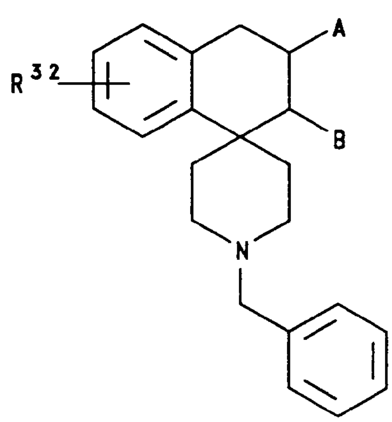

( I I A )

wherein A and B are as defined in Claim 6; and $R^{32}$ represents $C_{1-6}$ alkyl, halogen, cyano, trifluoromethyl, nitro or hydroxy.

**8.** A process as claimed in Claim 6 for the preparation of a compound represented by formula IIB and salts thereof:

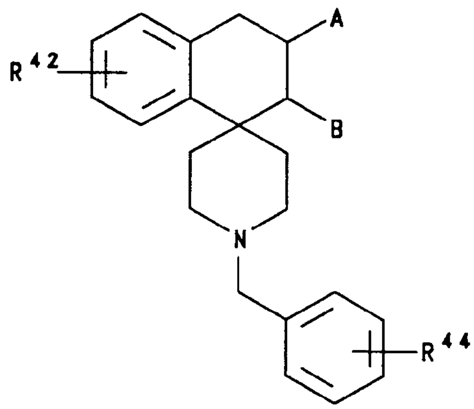

(IIB)

wherein A and B are as defined in Claim 6; $R^{42}$ represents hydrogen, $C_{1-6}$ alkyl, halogen, cyano, trifluoromethyl, nitro, hydroxy or $C_{1-6}$ alkoxy; and $R^{44}$ represents $C_{1-6}$ alkyl, halogen, cyano, trifluoromethyl, nitro or $C_{1-6}$ alkoxy.

**9.** A process as claimed in Claim 6 for the preparation of a compound represented by formula IIC and salts thereof:

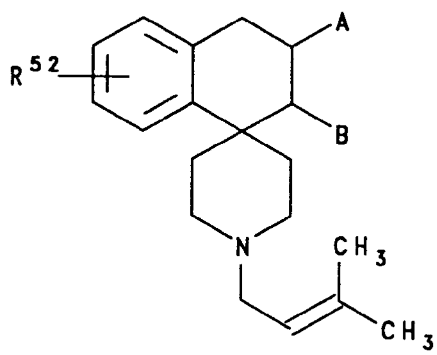

(IIC)

wherein A and B are as defined in Claim 6; and $R^{52}$ represents hydrogen, $C_{1-6}$ alkyl, halogen, cyano, trifluoromethyl, nitro, hydroxy or $C_{1-6}$ alkoxy.

**10.** A process as claimed in Claim 6 for the preparation of a compound represented by formula IID and salts thereof:

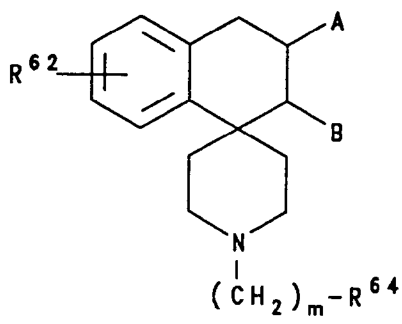

(IID)

wherein A and B are as defined in Claim 6; m is 1, 2 or 3; $R^{64}$ is optionally substituted $C_{3-7}$ cycloalkyl; and $R^{62}$ represents hydrogen, $C_{1-6}$ alkyl, halogen, cyano, trifluoromethyl, nitro, hydroxy or $C_{1-6}$ alkoxy.

**11.** A process as claimed in Claim 6 for the preparation of a compound represented by formula IIE and salts thereof:

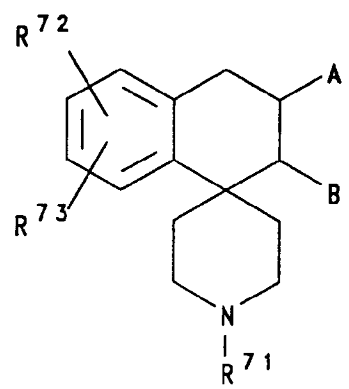

(IIE)

wherein A and B are as defined in Claim 6; $R^{71}$ represents $C_{4-6}$ alkenyl, $C_{3-7}$ heterocycloalkyl($C_{1-6}$)alkyl or heteroaryl($C_{1-6}$)alkyl, any of which groups may be optionally substituted; and $R^{72}$ and $R^{73}$ independently represent hydrogen, $C_{1-6}$ alkyl, halogen, cyano, trifluoromethyl, nitro, hydroxy or $C_{1-6}$ alkoxy.

**12.** A process as claimed in Claim 6 for the preparation of a compound selected from:
1'-(2"-picolyl)spiro[1,2,3,4-tetrahydronaphthalene-1,4'-piperidine];
1'-(4"-methoxybenzyl)spiro[1,2,3,4-tetrahydronaphthalene-1,4'-piperidine];
1'-(4"-methylbenzyl)spiro[1,2,3,4-tetrahydronaphthalene-1,4'-piperidine];
1'-benzylspiro[1,4-dihydro-7-methoxynaphthalene-1,4'-piperidine];
1'-cyclohexylmethylspiro[1,2,3,4-tetrahydronaphthalene-1,4'-piperidine];
1'-(4"-nitrobenzyl)spiro[1,2,3,4-tetrahydronaphthalene-1,4'-piperidine];
1'-(4"-chlorobenzyl)spiro[1,2,3,4-tetrahydronaphthalene-1,4'-piperidine];
1'-(2"-tetrahydrofurylmethyl)spiro[1,2,3,4-tetrahydronaphthalene-1,4'-piperidine];
1'-but-3"-enylspiro[1,2,3,4-tetrahydronaphthalene-1,4'-piperidine];
1'-cyclopropylmethylspiro[1,2,3,4-tetrahydronaphthalene-1,4'-piperidine];
1'-(3"-methylbut-2"-enyl)spiro[1,2,3,4-tetrahydronaphthalene-1,4'-piperidine];
1'-(2"-furylmethyl)spiro[1,2,3,4-tetrahydronaphthalene-1,4'-piperidine];
1'-(2"-thienylmethyl)spiro[1,2,3,4-tetrahydronaphthalene-1,4'-piperidine];
1'-benzylspiro[6-chloro-[1,2,3,4-tetrahydronaphthalene-1,4'-piperidine];
1'-benzylspiro[6-methyl-[1,2,3,4-tetrahydronaphthalene-1,4'-piperidine];
1'-benzylspiro[7-hydroxy-[1,2,3,4-tetrahydronaphthalene-1,4'-piperidine];
and salts thereof.

13. A process as claimed in Claim 1 for the preparation of a compound selected from:
1'-(n-butyl)spiro[1,2,3,4-tetrahydronaphthalene-1,4'-piperidine];
1'-benzylspiro[7-methoxy-[1,2,3,4-tetrahydronaphthalene-1,4'-piperidine];
1'-(2"-phenylethyl)spiro[1,2,3,4-tetrahydronaphthalene-1,4'-piperidine];
1'-n-hexylspiro[1,2,3,4-tetrahydronaphthalene-1,4'-piperidine];
1'-prop-2"-enylspiro[1,2,3,4-tetrahydronaphthalene-1,4'-piperidine];
and salts thereof.

14. A process for the preparation of a pharmaceutical composition which comprises mixing a compound prepared as claimed in Claim 13 or a pharmaceutically acceptable salt thereof with one or more pharmaceutically acceptable carriers and/or excipients.

**Patentansprüche**

**Patentansprüche für folgende Vertragsstaaten : AT, BE, CH, DE, DK, FR, GB, IT, LI, LU, NL, SE**

1. Verwendung einer Verbindung der Formel I oder eines pharmazeutisch annehmbaren Salzes davon:

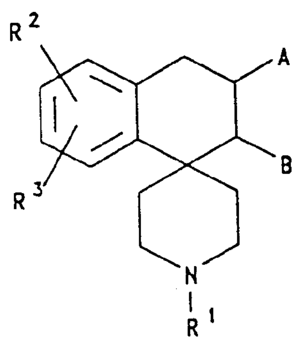

(I)

wobei

A und B jeweils Wasserstoff bedeuten oder A und B zusammen eine chemische Bindung darstellen,
$R^1$ Kohlenwasserstoff bedeutet,
$R^2$ und $R^3$ unabhängig Wasserstoff, Kohlenwasserstoff, Halogen, Cyano, Trifluormethyl, Nitro, $-OR^x$, $-SR^x$, $-NR^xR^y$, $-CO_2R^x$ oder $-CONR^xR^y$ oder zusammen Methylendioxy bedeuten und
$R^x$ und $R^y$ unabhängig Wasserstoff oder Kohlenwasserstoff bedeuten, wobei der Ausdruck "Kohlenwasserstoff", wie er hier verwendet wird, geradkettige, verzweigte und cyclische Gruppen umfaßt, einschließlich heterocyclische Gruppen, enthaltend bis zu 18 Kohlenstoffatome,

zur Herstellung eines Arzneimittels zur Behandlung und/oder Verhütung von psychiatrischen Störungen.

2. Verwendung nach Anspruch 1, wobei die Verbindung der Formel I ausgewählt ist aus 1'-Benzylspiro[1,2,3,4-tetrahydronaphthalin-1,4'-piperidin] und pharmazeutisch annehmbaren Salzen davon.

3. Pharmazeutisches Mittel, umfassend eine Verbindung der Formel IA oder ein pharmazeutisch verträgliches Salz davon

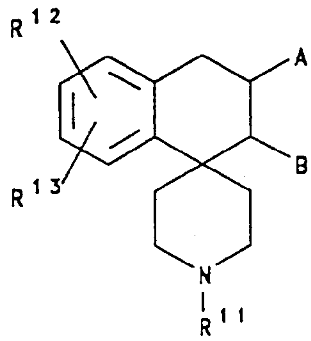

(IA)

wobei

A und B jeweils Wasserstoff bedeuten oder A und B zusammen eine chemische Bindung darstellen,

$R^{11}$ Kohlenwasserstoff bedeutet,

$R^{12}$ und $R^{13}$ unabhängig Wasserstoff, Kohlenwasserstoff, Halogen, Cyano, Trifluormethyl, Nitro, $-OR^x$, $-SR^x$, $-NR^xR^y$, $-CO_2R^x$ oder $-CONR^xR^y$ oder zusammen Methylendioxy bedeuten und

$R^x$ und $R^y$ unabhängig Wasserstoff oder Kohlenwasserstoff bedeuten,

mit der Maßgabe, daß, wenn A und B beide Wasserstoff bedeuten und $R^{11}$ Alkyl, Allyl, Aryl, Aralkyl, Cycloalkyl oder Ar(cycloalkyl) bedeutet, $R^{12}$ und $R^{13}$ nicht Wasserstoff, Alkoxy oder Hydroxyalkyl bedeuten,

mit der weiteren Maßgabe, daß, wenn A, B und $R^{12}$ jeweils Wasserstoff bedeuten und $R^{11}$ eine Gruppe ist der Formel $-CH_2CH_2CX^1Ph_2$, in der $X^1$ Cyano oder $C_2$-$C_7$-Alkylcarbonyl bedeutet, $R^{13}$ nicht Wasserstoff oder Methyl in 7-Stellung der Tetrahydronaphthalin-Einheit ist,

mit der weiteren Maßgabe, daß, wenn $R^{11}$ eine Gruppe ist der Formel $-CH_2CH_2CX^2Ph_2$, in der $X^2$ Dimethylaminocarbonyl, 1-Hydroxypropyl oder 1-Acetoxypropyl bedeutet, A, B, $R^{12}$ und $R^{13}$ nicht gleichzeitig Wasserstoff bedeuten, wobei der Ausdruck "Kohlenwasserstoff", wie er hier verwendet wird, geradkettige, verzweigte und cyclische Gruppen umfaßt, einschließlich heterocyclische Gruppen, enthaltend bis zu 18 Kohlenstoffatome,

zusammen mit einem oder mehreren pharmazeutisch annehmbaren Trägern und/oder Exzipientien.

4. Pharmazeutisches Mittel nach Anspruch 3, wobei der Wirkstoff 1'-Benzylspiro[1,4-dihydronaphthalin-1,4'-piperidin] oder ein pharmazeutisch annehmbares Salz davon ist.

5. Verbindung der Formel IA, wie in Anspruch 3 definiert, oder ein pharmazeutisch annehmbares Salz davon zur Verwendung bei der Therapie.

6. Verbindung der Formel II oder ein Salz davon

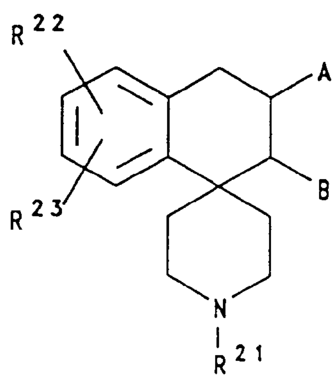

(II)

wobei

A und B jeweils Wasserstoff bedeuten oder A und B zusammen eine chemische Bindung darstellen,

$R^{21}$ Kohlenwasserstoff bedeutet, wobei der Ausdruck "Kohlenwasserstoff", wie er hier verwendet wird, geradkettige, verzweigte und cyclische Gruppen umfaßt, einschließlich heterocyclische Gruppen, enthaltend bis zu 18 Kohlenstoffatome,

$R^{22}$ und $R^{23}$ unabhängig Wasserstoff, Kohlenwasserstoff, Halogen, Cyano, Trifluormethyl, Nitro, $-OR^x$, $-SR^x$, $-NR^xR^y$, $-CO_2R^x$ oder $-CONR^xR^y$ oder zusammen Methylendioxy bedeuten und

$R^x$ und $R^y$ unabhängig Wasserstoff oder Kohlenwasserstoff bedeuten,

mit der Maßgabe, daß, wenn A und B beide Wasserstoff bedeuten und $R^{21}$ Alkyl, Allyl, Aryl, Aralkyl, Cycloalkyl oder Ar(cycloalkyl) bedeutet, $R^{22}$ und $R^{23}$ nicht Wasserstoff, Alkoxy oder Hydroxyalkyl bedeuten,

mit der weiteren Maßgabe, daß, wenn A, B und $R^{22}$ jeweils Wasserstoff bedeuten und $R^{21}$ Methyl ist, $R^{23}$ nicht Hydroxy in 6-Stellung der Tetrahydronaphthalin-Einheit bedeutet,

mit der weiteren Maßgabe, daß, wenn A, B und $R^{22}$ jeweils Wasserstoff bedeuten und $R^{21}$ Benzyl ist, $R^{23}$ nicht Methyl in 7-Stellung der Tetrahydronaphthalin-Einheit bedeutet,

mit der weiteren Maßgabe, daß, wenn A, B und $R^{22}$ jeweils Wasserstoff bedeuten und $R^{21}$ eine Gruppe ist der Formel $-CH_2CH_2CX^1Ph_2$, in der $X^1$ Cyano oder $C_2$-$C_7$-Alkylcyrbonyl bedeutet, $R^{23}$ nicht Wasserstoff oder Methyl in 7-Stellung der Tetrahydronaphthalin-Einheit bedeutet,

mit der weiteren Maßgabe, daß, wenn $R^{21}$ eine Gruppe ist der Formel $-CH_2CH_2CX^2Ph_2$, in der $X^2$ Dimethylaminocarbonyl, 1-Hydroxypropyl oder 1-Acetoxypropyl bedeutet, A, B, $R^{22}$ und $R^{23}$ nicht gleichzeitig Wasserstoff bedeuten,

mit der weiteren Maßgabe, daß, wenn A und B zusammen eine chemische Bindung darstellen und $R^{21}$ Benzyl ist, $R^{22}$ und $R^{23}$ nicht gleichzeitig Wasserstoff bedeuten.

7. Verbindung nach Anspruch 6, angegeben durch die allgemeine Formel IIA und Salze davon

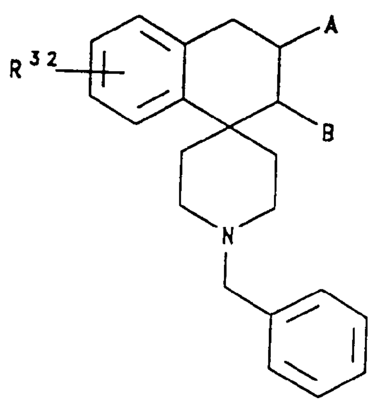

(IIA)

wobei A und B wie in Anspruch 6 definiert sind und $R^{32}$ $C_1$-$C_6$-Alkyl, Halogen, Cyano, Trifluormethyl, Nitro oder Hydroxy bedeutet.

8. Verbindung nach Anspruch 6, angegeben durch die allgemeine Formel IIB und Salze davon

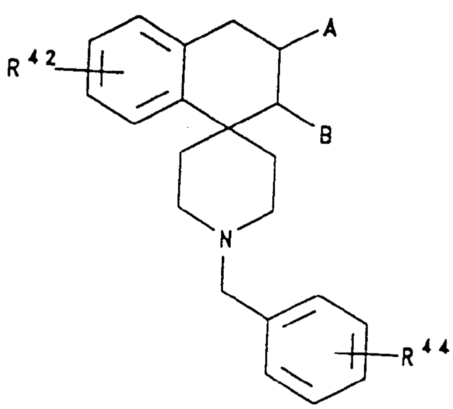

(IIB)

wobei A und B wie in Anspruch 6 definiert sind und $R^{42}$ Wasserstoff, $C_1$-$C_6$-Alkyl, Halogen, Cyano, Trifluormethyl, Nitro, Hydroxy oder $C_1$-$C_6$-Alkoxy bedeutet und $R^{44}$ $C_1$-$C_6$-Alkyl, Halogen, Cyano, Trifluormethyl, Nitro, oder $C_1$-$C_6$-Alkoxy bedeutet.

9. Verbindung nach Anspruch 6, angegeben durch die allgemeine Formel IIC und Salze davon

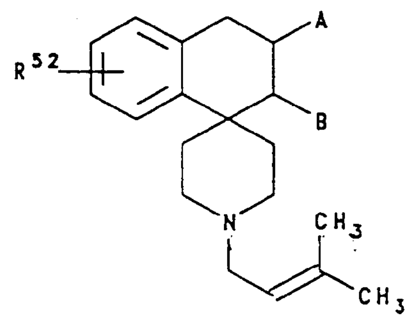

(IIC)

wobei A und B wie in Anspruch 6 definiert sind und $R^{52}$ Wasserstoff, $C_1$-$C_6$-Alkyl, Halogen, Cyano, Trifluormethyl, Nitro, Hydroxy oder $C_1$-$C_6$-Alkoxy bedeutet.

10. Verbindung nach Anspruch 6, angegeben durch die allgemeine Formel IID und Salze davon

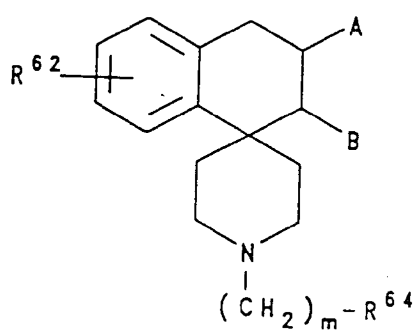

(IID)

wobei A und B wie in Anspruch 6 definiert sind, m 1, 2 oder 3 ist und $R^{64}$ gegebenenfalls substituiertes $C_3$-$C_7$-Cycloalkyl bedeutet und $R^{62}$ Wasserstoff, $C_1$-$C_6$-Alkyl, Halogen, Cyano, Trifluormethyl, Nitro, Hydroxy oder $C_1$-$C_6$-Alkoxy bedeutet.

11. Verbindung nach Anspruch 6, angegeben durch die allgemeine Formel IIE und Salze davon

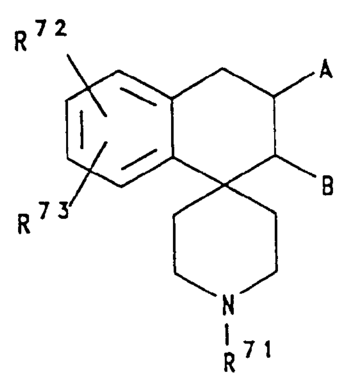

(IIE)

wobei A und B wie in Anspruch 6 definiert sind, $R^{71}$ $C_4$-$C_6$-Alkenyl, $C_3$-$C_7$-Heterocycloalkyl ($C_1$-$C_6$)alkyl oder Heteroaryl($C_1$-$C_6$)alkyl bedeutet, wobei jede dieser Gruppen gegebenenfalls substituiert sein kann, und $R^{72}$ und $R^{73}$ unabhängig Wasserstoff, $C_1$-$C_6$-Alkyl, Halogen,Cyano, Trifluormethyl, Nitro, Hydroxy oder $C_1$-$C_6$-Alkoxy bedeuten.

12. Verbindung nach Anspruch 6, ausgewählt aus
1'-(2"-Picolyl)spiro[1,2,3,4-tetrahydronaphthalin,1,4'-piperidin],

1'-(4"-Methoxybenzyl)spiro[1,2,3,4-tetrahydronaphthalin,1,4'-piperidin],
1'-(4"-Methylbenzyl)spiro[1,2,3,4-tetrahydronaphthalin,1,4'-piperidin],
1'-Benzlspiro[1,4-dihydro-7-methoxynaphthalin, 1,4'-piperidin],
1'-Cyclohexylmethylspiro[1,2,3,4-tetrahydronaphthalin,1,4'-piperidin],
1'-(4"-Nitrobenzyl)spiro[1,2,3,4-tetrahydronaphthalin,1,4'-piperidin],
1'-(4"-Chlorbenzyl)spiro[1,2,3,4-tetrahydronaphthalin,1,4'-piperidin]
1'-(2"-Tetrahydrofurylmethyl)spiro[1,2,3,4-tetrahydronaphthalin-1,4'-piperidin],
1'-But-3"-enylspiro[1,2,3,4-tetrahydronaphthalin-1,4'-piperidin],
1'-Cyclopropylmethylspiro[1,2,3,4-tetrahydronaphthalin-1,4'-piperidin]
1'-(3'-Methylbut-2"-enyl)spiro[1,2,3,4-tetrahydronaphthalin-1,4'-piperidin],
1'-(2"-Furylmethyl)spiro[1,2,3,4-tetrahydronaphthalin-1,4'-piperidin],
1'-(2"-Thienylmethyl)spiro[1,2,3,4-tetrahydronaphthalin-1,4'-piperidin],
1'-Benzylspiro[6-chlor-1,2,3,4-tetrahydronaphthalin-1,4'-piperidin],
1'-Benzylspiro[6-methyl-1,2,3,4-tetrahydronaphthalin-1,4'-piperidin],
1'-Benzylspiro[7-hydroxy-1,2,3,4-tetrahydronaphthalin-1,4'-piperidin]
und Salze davon.

**13.** Verbindung ausgewählt aus
1'-(n-Butyl)spiro[1,2,3,4-tetrahydronaphthalin-1,4'-piperidin],
1'-Benzylspiro[7-methoxy-1,2,3,4-tetrahydronaphthalin-1,4'-piperidin]
1'-(2"-Phenylethyl)spiro[1,2,3,4-tetrahydronaphthalin-1,4'-piperidin],
1'-n-Hexylspiro[1,2,3,4-tetrahydronaphthalin-1,4'-piperidin],
1'-Prop-2"-enylspiro[1,2,3,4-tetrahydronaphthalin-1,4'-piperidin]
und Salze davon.

**14.** Pharmazeutisches Mittel, umfassend eine Verbindung nach Anspruch 13 oder ein pharmazeutisch annehmbares Salz davon zusammen mit einem oder mehreren pharmazeutisch annehmbaren Trägern und/oder Exzipientien.

**15.** Verbindung nach Anspruch 13 oder ein pharmazeutisch annehmbares Salz davon zur Verwendung bei der Therapie.

**16.** Verfahren zur Herstellung einer Verbindung der Formel I, wie in Anspruch 1 definiert, wobei das Verfahren umfaßt:

(A) zur Herstellung einer Verbindung der Formel I, bei der A und B jeweils Wasserstoff bedeuten:

Reduktion einer Verbindung der Formel III:

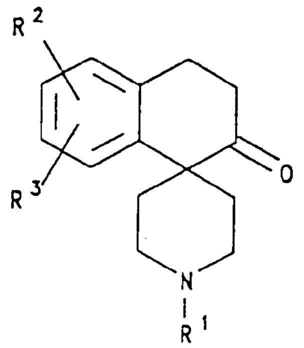

(III)

wobei $R^1$, $R^2$ und $R^3$ wie in Anspruch 1 definiert sind, oder

(B) zur Herstellung einer Verbindung der Formel I, bei der A und B zusammen eine chemische Bindung darstellen:

Dehydratisierung einer Verbindung der Formel V:

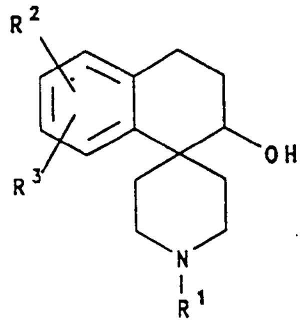

(V)

wobei $R^1$, $R^2$ und $R^3$ wie in Anspruch 1 definiert sind, oder

(C) Umsetzung einer Verbindung der Formel $R^1$-L mit einer Verbindung der Formel VI:

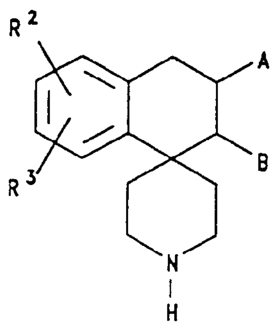

(VI)

wobei A, B, $R^1$, $R^2$ und $R^3$ wie in Anspruch 1 definiert sind und L eine austretende Gruppe ist.

**Patentansprüche für folgende Vertragsstaaten : ES, GR**

1. Verfahren zur Herstellung einer Verbindung der Formel I oder eines pharmazeutisch annehmbaren Salzes davon:

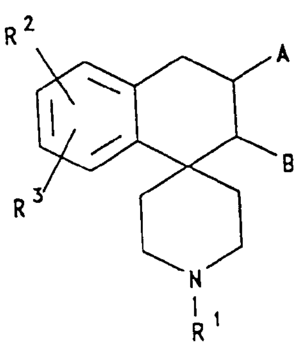

(I)

wobei

A und B jeweils Wasserstoff bedeuten oder A und B zusammen eine chemische Bindung darstellen,

$R^1$ Kohlenwasserstoff bedeutet,

$R^2$ und $R^3$ unabhängig Wasserstoff, Kohlenwasserstoff, Halogen, Cyano, Trifluormethyl, Nitro, $-OR^x$, $-SR^x$, $-NR^xR^y$, $-CO_2R^x$ oder $-CONR^xR^y$ oder zusammen Methylendioxy bedeuten und

$R^x$ und $R^y$ unabhängig Wasserstoff, Kohlenwasserstoff bedeuten, wobei der Ausdruck "Kohlenwasserstoff", wie er hier verwendet wird, geradkettige, verzweigte und cyclische Gruppen umfaßt, einschließlich heterocyclische Gruppen, enthaltend bis zu 18 Kohlenstoffatome, wobei das Verfahren umfaßt:

(A) zur Herstellung einer Verbindung der Formel I, bei der A und B jeweils Wasserstoff bedeuten:

Reduktion einer Verbindung der Formel III:

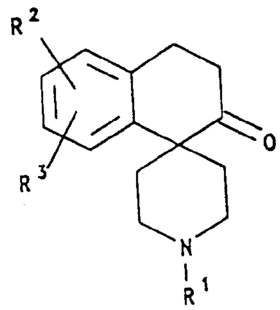

(III)

wobei $R^1$, $R^2$ und $R^3$ wie in Anspruch 1 definiert sind, oder

(B) zur Herstellung einer Verbindung der Formel I, bei der A und B zusammen eine chemische Bindung darstellen:

Dehydratisierung einer Verbindung der Formel V:

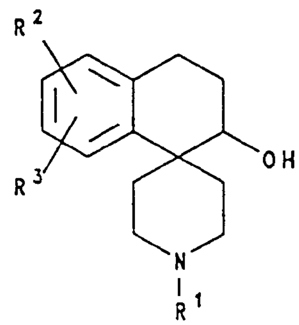

(V)

wobei $R^1$, $R^2$ und $R^3$ wie in Anspruch 1 definiert sind, oder

(C) Umsetzung einer Verbindung der Formel $R^1$-L mit einer Verbindung der Formel VI:

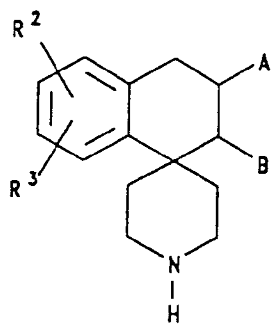

(VI)

wobei A, B, $R^1$, $R^2$ und $R^3$ wie in Anspruch 1 definiert sind und L eine austretende Gruppe ist.

2. Verwendung einer Verbindung der Formel I, wie in Anspruch 1, angegeben, zur Herstellung eines Arzneimittels zur Behandlung und/oder Verhütung von psychiatrischen Störungen.

3. Verwendung nach Anspruch 2, wobei die Verbindung der Formel I ausgewählt ist aus 1'-Benzylspiro[1,2,3,4-tetrahydronaphthalin-1,4'-piperidin] und pharmazeutisch annehmbaren Salzen davon.

4. Verfahren zur Herstellung eines pharmazeutisches Mittels, umfassend das Vermischen einer Verbindung der Formel IA oder eines pharmazeutisch verträglichen Salzes davon

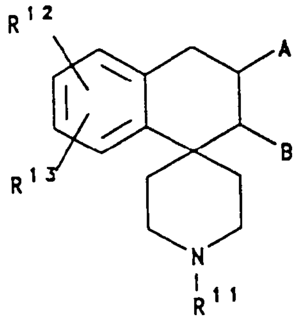

(IA)

wobei

A und B jeweils Wasserstoff bedeuten oder A und B zusammen eine chemische Bindung darstellen,

$R^{11}$ Kohlenwasserstoff bedeutet,

$R^{12}$ und $R^{13}$ unabhängig Wasserstoff, Kohlenwasserstoff, Halogen, Cyano, Trifluormethyl, Nitro, $-OR^x$, $-SR^x$, $-NR^xR^y$, $-CO_2R^x$ oder $-CONR^xR^y$ oder zusammen Methylendioxy bedeuten und

$R^x$ und $R^y$ unabhängig Wasserstoff oder Kohlenwasserstoff bedeuten,

mit der Maßgabe, daß, wenn A und B beide Wasserstoff bedeuten und $R^{11}$ Alkyl, Allyl, Aryl, Aralkyl, Cycloalkyl oder Ar(cycloalkyl) bedeutet, $R^{12}$ und $R^{13}$ nicht Wasserstoff, Alkoxy oder Hydroxyalkyl bedeuten,

mit der weiteren Maßgabe, daß, wenn A, B und $R^{12}$ jeweils Wasserstoff bedeuten und $R^{11}$ eine Gruppe ist der Formel $-CH_2CH_2CX^1Ph_2$, in der $X^1$ Cyano oder $C_2$-$C_7$-Alkylcarbonyl bedeutet, $R^{13}$ nicht Wasserstoff oder Methyl in 7-Stellung der Tetrahydronaphthalin-Einheit ist,

mit der weiteren Maßgabe, daß, wenn $R^{11}$ eine Gruppe ist der Formel $-CH_2CH_2CX^2Ph_2$, in der $X^2$ Dimethylaminocarbonyl, 1-Hydroxypropyl oder 1-Acetoxypropyl bedeutet, A, B, $R^{12}$ und $R^{13}$ nicht gleichzeitig Wasserstoff bedeuten, wobei der Ausdruck "Kohlenwasserstoff", wie er hier verwendet wird, geradkettige, verzweigte und cyclische Gruppen umfaßt, einschließlich heterocyclische Gruppen, enthaltend bis zu 18 Kohlenstoffatome,

mit einem oder mehreren pharmazeutisch annehmbaren Trägern und/oder Exzipientien.

5. Verfahren zur Herstellung eines pharmazeutischen Mittels, wobei der Wirkstoff 1'-Benzylspiro[1,4-dihydronaphthalin-1,4'-piperidin] oder ein pharmazeutisch annehmbares Salz davon ist.

6. Verfahren nach Anspruch 1 zur Herstellung einer Verbindung der Formel II oder eines Salzes davon

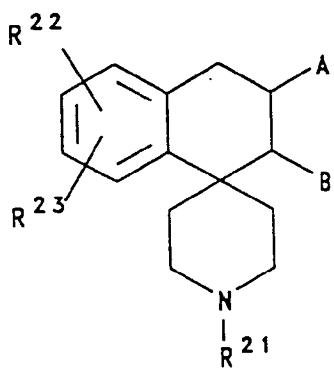

(II)

wobei

A und B jeweils Wasserstoff bedeuten oder A und B zusammen eine chemische Bindung darstellen,

$R^{21}$ Kohlenwasserstoff bedeutet, wobei der Ausdruck "Kohlenwasserstoff", wie er hier verwendet wird, geradkettige, verzweigte und cyclische Gruppen umfaßt, einschließlich heterocyclische Gruppen, enthaltend bis zu 18 Kohlenstoffatome,

$R^{22}$ und $R^{23}$ unabhängig Wasserstoff, Kohlenwasserstoff, Halogen, Cyano, Trifluormethyl, Nitro, $OR^x$, $-SR^x$, $-NR^xR^y$, $-CO_2R^x$ oder $-CONR^xR^y$ oder zusammen Methylendioxy bedeuten und

$R^x$ und $R^y$ unabhängig Wasserstoff oder Kohlenwasserstoff bedeuten,

mit der Maßgabe, daß, wenn A und B beide Wasserstoff bedeuten und $R^{21}$ Alkyl, Allyl, Aryl, Aralkyl,

Cycloalkyl oder Ar(cycloalkyl) bedeutet, $R^{22}$ und $R^{23}$ nicht Wasserstoff, Alkoxy oder Hydroxyalkyl bedeuten,

mit der weiteren Maßgabe, daß, wenn A, B und $R^{22}$ jeweils Wasserstoff bedeuten und $R^{21}$ Methyl ist, $R^{23}$ nicht Hydroxy in 6-Stellung der Tetrahydronaphthalin-Einheit bedeutet,

mit der weiteren Maßgabe, daß, wenn A, B und $R^{22}$ jeweils Wasserstoff bedeuten und $R^{21}$ Benzyl ist, $R^{23}$ nicht Methyl in 7-Stellung der Tetrahydronaphthalin-Einheit bedeutet,

mit der weiteren Maßgabe, daß, wenn A, B und $R^{22}$ jeweils Wasserstoff bedeuten und $R^{21}$ eine Gruppe ist der Formel $-CH_2CH_2CX^1Ph_2$, in der $X^1$ Cyano oder $C_2$-$C_7$-Alkylcarbonyl bedeutet, $R^{23}$ nicht Wasserstoff oder Methyl in 7-Stellung der Tetrahydronaphthalin-Einheit bedeutet,

mit der weiteren Maßgabe, daß, wenn $R^{21}$ eine Gruppe ist der Formel $-CH_2CH_2CX^2Ph_2$, in der $X^2$ Dimethylaminocarbonyl, 1-Hydroxypropyl oder 1-Acetoxypropyl bedeutet, A, B, $R^{22}$ und $R^{23}$ nicht gleichzeitig Wasserstoff bedeuten,

mit der weiteren Maßgabe, daß, wenn A und B zusammen eine chemische Bindung darstellen und $R^{21}$ Benzyl ist, $R^{22}$ und $R^{23}$ nicht gleichzeitig Wasserstoff bedeuten.

7. Verfahren nach Anspruch 6 zur Herstellung einer Verbindung, angegeben durch die allgemeine Formel IIA und Salzen davon

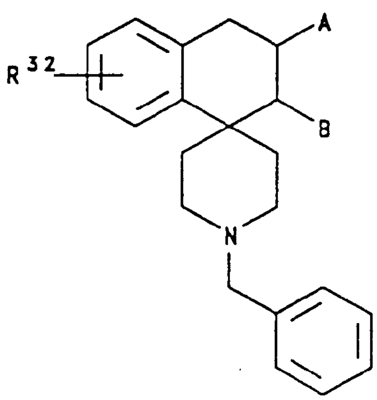

(IIA)

wobei A und B wie in Anspruch 6 definiert sind und $R^{32}$ $C_1$-$C_6$-Alkyl, Halogen, Cyano, Trifluormethyl, Nitro oder Hydroxy bedeutet.

8. Verfahren nach Anspruch 6 zur Herstellung einer Verbindung, angegeben durch die allgemeine Formel IIB und Salzen davon

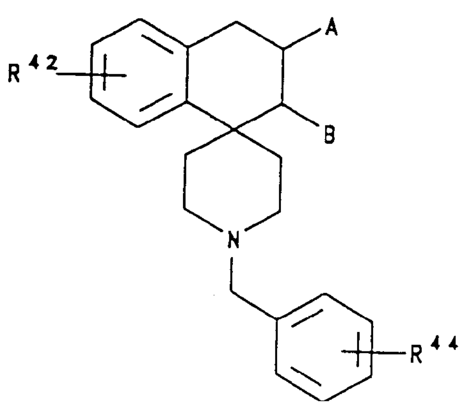

(IIB)

wobei A und B wie in Anspruch 6 definiert sind und $R^{42}$ Wasserstoff, $C_1$-$C_6$-Alkyl, Halogen, Cyano, Trifluormethyl, Nitro, Hydroxy oder $C_1$-$C_6$-Alkoxy bedeutet und $R^{44}$ $C_1$-$C_6$-Alkyl, Halogen, Cyano, Trifluormethyl, Nitro, oder $C_1$-$C_6$-Alkoxy bedeutet.

9. Verfahren nach Anspruch 6 zur Herstellung einer Verbindung, angegeben durch die allgemeine Formel IIC und Salze davon

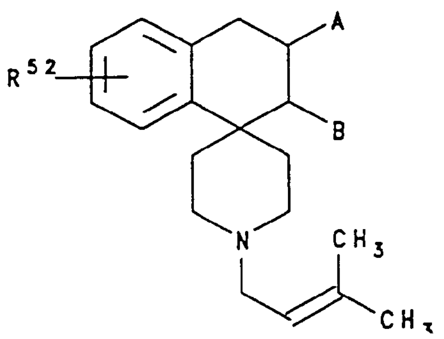

(IIC)

wobei A und B wie in Anspruch 6 definiert sind $R^{52}$ Wasserstoff, $C_1$-$C_6$-Alkyl, Halogen, Cyano, Trifluormethyl, Nitro, Hydroxy oder $C_1$-$C_6$-Alkoxy bedeutet.

**10.** Verfahren nach Anspruch 6 zur Herstellung einer Verbindung, angegeben durch die allgemeine Formel IID und Salze davon

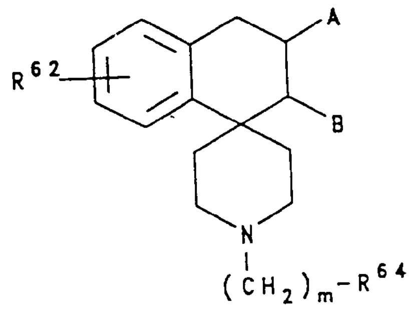

(IID)

wobei A und B wie in Anspruch 6 definiert sind, m 1, 2 oder 3 ist und $R^{64}$ gegebenenfalls substituiertes $C_3$-$C_7$-Cycloalkyl bedeutet und $R^{62}$ Wasserstoff, $C_1$-$C_6$-Alkyl, Halogen, Cyano, Trifluormethyl, Nitro, Hydroxy oder $C_1$-$C_6$-Alkoxy bedeutet.

**11.** Verfahren nach Anspruch 6 zur Herstellung einer Verbindung, angegeben durch die allgemeine Formel IIE und Salze davon

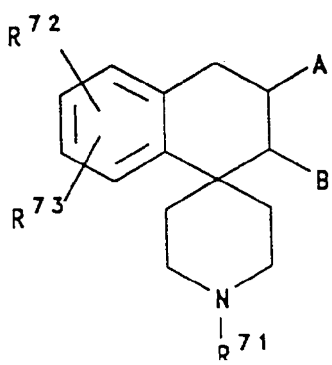

(IIE)

wobei A und B wie in Anspruch 6 definiert sind, $R^{71}$ $C_4$-$C_6$-Alkenyl, $C_3$-$C_7$-Heterocycloalkyl($C_1$-$C_6$)alkyl oder Heteroaryl($C_1$-$C_6$)alkyl bedeutet, wobei jede dieser Gruppen gegebenenfalls substituiert sein kann, und $R^{72}$ und $R^{73}$ unabhängig Wasserstoff, $C_1$-$C_6$-Alkyl, Halogen,Cyano, Trifluormethyl, Nitro, Hydroxy oder $C_1$-$C_6$-Alkoxy bedeuten.

**12.** Verfahren nach Anspruch 6 zur Herstellung einer Verbindung, ausgewählt aus
1'-(2"-Picolyl)spiro[1,2,3,4-tetrahydronaphthalin,1,4'-piperidin],
1'-(4"-Methoxybenzyl)spiro[1,2,3,4-tetrahydronaphthalin,1,4'-piperidin],
1'-(4"-Methylbenzyl)spiro[1,2,3,4-tetrahydronaphthalin,1,4'-piperidin],

1'-Benzylspiro[1,4-dihydro-7-methoxynaphthalin,1,4'-piperidin],
1'-Cyclohexylmethylspiro[1,2,3,4-tetrahydronaphthalin,1,4'-piperidin],
1'-(4"-Nitrobenzyl)spiro[1,2,3,4-tetrahydronaphthalin,1,4'-piperidin],
1'-(4"-Chlorbenzyl)spiro[1,2,3,4-tetrahydronaphthalin,1,4'-piperidin],
1'-(2"-Tetrahydrofurylmethyl)spiro[1,2,3,4-tetrahydronaphthalin- 1,4'-piperidin],
1'-But-3"-enylspiro[1,2,3,4-tetrahydronaphthalin-1,4'-piperidin],
1'-Cyclopropylmethylspiro[1,2,3,4-tetrahydronaphthalin-1,4'-piperidin],
1'-(3"-Methylbut-2"-enyl)spiro[1,2,3,4-tetrahydronaphthalin-1,4'-piperidin],
1'-(2"-Furylmethyl)spiro[1,2,3,4-tetrahydronaphthalin-1,4'-piperidin],
1'-(2"-Thienylmethyl)spiro[1,2,3,4-tetrahydronaphthalin-1,4'-piperidin],
1'-Benzylspiro[6-chlor-1,2,3,4-tetrahydronaphthalin-1,4'-piperidin],
1'-Benzylspiro[6-methyl-1,2,3,4-tetrahydronaphthalin-1,4'-piperidin],
1'-Benzylspiro[7-hydroxy-1,2,3,4-tetrahydronaphthalin-1,4'-piperidin]
und Salze davon.

**13.** Verfahren nach Anspruch 1 zur Herstellung einer Verbindung, ausgewählt aus
1'-(n-Butyl)spiro[1,2,3,4-tetrahydronaphthalin-1,4'-piperidin], 1'-Benzylspiro[7-methoxy-1,2,3,4-tetrahydronaphthalin-1,4'-piperidin],
1'-(2"-Phenylethyl)spiro[1,2,3,4-tetrahydronaphthalin-1,4'-piperidin],
1'-n-Hexylspiro[1,2,3,4-tetrahydronaphthalin-1,4'-piperidin],
1'-Prop-2"-enylspiro[1,2,3,4-tetrahydronaphthalin-1,4' -piperidin]
und Salze davon.

**14.** Verfahren zur Herstellung eines pharmazeutischen Mittels, umfassend das Vermischen einer Verbindung nach Anspruch 13 oder eines pharmazeutisch annehmbaren Salzes davon mit einem oder mehreren pharmazeutisch annehmbaren Trägern und/oder Exzipientien.

## Revendications

### Revendications pour les Etats contractants suivants : AT, BE, CH, DE, DK, FR, IT, LI, LU, NL, SE

**1.** Utilisation d'un composé de formule I ou d'un sel pharmaceutiquement acceptable de celui-ci:

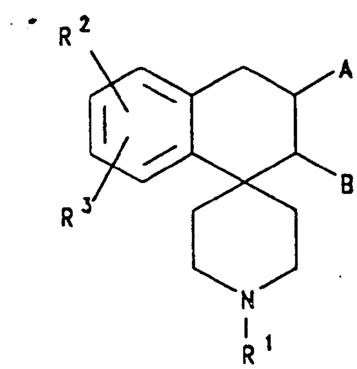

( I )

où

A et B représentent chacun l'hydrogène, ou A et B représentent ensemble une liaison chimique;

$R^1$ représente un groupe hydrocarboné;

$R^2$ et $R^3$ représentent, indépendamment l'un de l'autre, l'hydrogène ou un groupe hydrocarboné, halogène, cyano, trifluorométhyle, nitro, $-OR^x$, $-SR^x$, $-NR^xR^y$, $-CO_2R^x$ ou $-CONR^xR^y$, ou représentent ensemble un groupe méthylènedioxy; et

$R^x$ et $R^y$ représentent, indépendamment l'un de l'autre, l'hydrogène ou un groupe hydrocarboné;

le terme "hydrocarboné" tel qu'il est présentement utilisé comprenant des groupes à chaîne droite, ramifiés et cycliques, y compris des groupes hétérocycliques, contenant jusqu'à 18 atomes de carbone; pour la préparation d'un médicament pour le traitement et/ou la prévention des troubles psychiatriques.

2. Utilisation selon la revendication 1, dans laquelle le composé de formule I est choisi parmi: la 1'-benzylspiro[1,2,3,4-tétrahydronaphtalène-1,4'-pipéridine] et des sels pharmaceutiquement acceptables de celle-ci.

3. Composition pharmaceutique comprenant un composé de formule IA ou un sel pharmaceutiquement acceptable de ce composé:

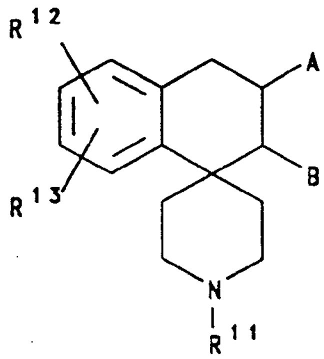

(IA)

où

A et B représentent chacun l'hydrogène, ou A et B représentent ensemble une liaison chimique;

$R^{11}$ représente un groupe hydrocarboné;

$R^{12}$ et $R^{13}$ représentent, indépendamment l'un de l'autre, l'hydrogène ou un groupe hydrocarboné, halogène, cyano, trifluorométhyle, nitro, $-OR^x$, $-SR^x$, $-NR^xR^y$, $-CO_2R^x$ ou $-CONR^xR^y$, ou représentent ensemble un groupe méthylènedioxy; et

$R^x$ et $R^y$ représentent, indépendamment l'un de l'autre, l'hydrogène ou un groupe hydrocarboné;

à la condition que, lorsque A et B représentent tous les deux l'hydrogène et que $R^{11}$ représente un groupe alkyle, allyle, aryle, aralkyle, cycloalkyle ou ar(cycloalkyle), alors $R^{12}$ et $R^{13}$ ne représentent pas l'hydrogène ou un groupe alcoxy ou hydroxyalkyle;

à la condition également que, lorsque A, B et $R^{12}$ représentent chacun l'hydrogène et que $R^{11}$ est un groupe de formule $-CH_2CH_2CX^1Ph_2$ dans laquelle $X^1$ représente un groupe cyano ou alkyl$(C_{2-7})$carbonyle, alors $R^{13}$ ne représente pas l'hydrogène ou un groupe méthyle sur la position 7 de la partie tétrahydronaphtalène;

à la condition en outre que, lorsque $R^{11}$ est un groupe de formule $-CH_2CH_2CX^2Ph_2$ dans laquelle $X^2$ représen- te un groupe diméthylaminocarbonyle, 1-hydroxypropyle ou 1-acétoxypropyle, alors A, B, $R^{12}$ et $R^{13}$ ne représentent pas simultanément l'hydrogène;

le terme "hydrocarboné" tel qu'il est présentement utilisé comprenant des groupes à chaîne droite, ramifiés et cycliques, y compris des groupes hétérocycliques, contenant jusqu'à 18 atomes de carbone; en association avec un ou plusieurs supports et/ou excipients pharmaceutiquement acceptables.

4. Composition pharmaceutique selon la revendication 3, dans laquelle le constituant actif est la 1'-benzylspiro[1,4-dihydronaphtalène-1,4'-pipéridine] ou un sel pharmaceutiquement acceptable de celle-ci.

5. Composé de formule IA tel que défini dans la revendication 3 ou sel pharmaceutiquement acceptable de celui-ci pour utilisation en thérapie.

6. Composé de formule II ou sel de ce composé:

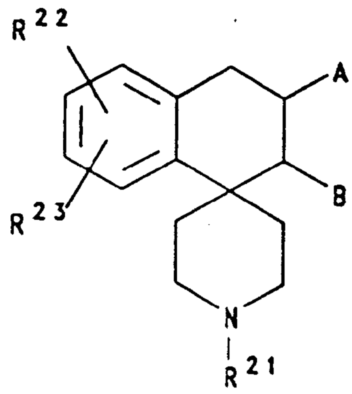

( I I )

où

A et B représentent chacun l'hydrogène, ou A et B représentent ensemble une liaison chimique;

$R^{21}$ représente un groupe hydrocarboné;

le terme "hydrocarboné" tel qu'il est présentement utilisé comprenant des groupes à chaîne droite, ramifiés et cycliques, y compris des groupes hétérocycliques, contenant jusqu'à 18 atomes de carbone;

$R^{22}$ et $R^{23}$ représentent, indépendamment l'un de l'autre, l'hydrogène ou un groupe hydrocarboné, halogène, cyano, trifluorométhyle, nitro, $-OR^x$, $-SR^x$, $-NR^xR^y$, $-CO_2R^x$ ou $-CONR^xR^y$, ou représentent ensemble un groupe méthylènedioxy; et

$R^x$ et $R^y$ représentent, indépendamment l'un de l'autre, l'hydrogène ou un groupe hydrocarboné;

à la condition que, lorsque A et B représentent tous les deux l'hydrogène et que $R^{21}$ représente un groupe alkyle, allyle, aryle, aralkyle, cycloalkyle ou ar(cycloalkyle), alors $R^{22}$ et $R^{23}$ ne représentent pas l'hydrogène ou un groupe alcoxy ou hydroxyalkyle;

à la condition également que, lorsque A, B et $R^{22}$ représentent chacun l'hydrogène et que $R^{21}$ est un groupe méthyle, alors $R^{23}$ ne représente pas un groupe hydroxy sur la position 6 de la partie tétrahydronaphtalène;

à la condition également que, lorsque A, B et $R^{22}$ représentent chacun l'hydrogène et que $R^{21}$ est un groupe benzyle, alors $R^{23}$ ne représente pas un groupe méthyle sur la position 7 de la partie tétrahydronaphtalène;

à la condition également que, lorsque A, B et $R^{22}$ représentent chacun l'hydrogène et que $R^{21}$ est un groupe de formule $-CH_2CH_2CX^1Ph_2$ dans laquelle $X^1$ représente un groupe cyano ou alkyl($C_{2-7}$)carbonyle, alors $R^{23}$ ne représente pas l'hydrogène ou un groupe méthyle sur la position 7 de la partie tétrahydronaphtalène;

à la condition également que, lorsque $R^{21}$ est un groupe de formule $-CH_2CH_2CX^2Ph_2$ dans laquelle $X^2$ représente un groupe diméthylaminocarbonyle, 1-hydroxypropyle ou 1-acétoxypropyle, alors A, B, $R^{22}$ et $R^{23}$ ne représentent pas simultanément l'hydrogène;

à la condition en outre que, lorsque A et B représentent ensemble une liaison chimique et que $R^{21}$ représente un groupe benzyle, alors $R^{22}$ et $R^{23}$ ne représentent pas simultanément l'hydrogène.

7. Composé selon la revendication 6 représenté par la formule IIA et les sels de ce composé:

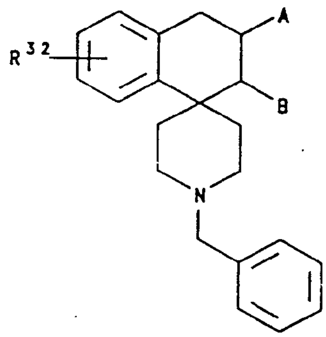

(IIA)

où A et B sont tels que définis dans la revendication 6; et $R^{32}$ représente un groupe alkyle en $C_{1-6}$, halogène, cyano, trifluorométhyle, nitro ou hydroxy.

8. Composé selon la revendication 6 représenté par la formule IIB et les sels de ce composé:

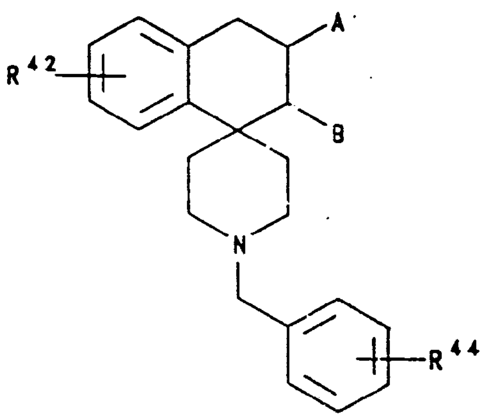

(IIB)

où A et B sont tels que définis dans la revendication 6; $R^{42}$ représente l'hydrogène ou un groupe alkyle en $C_{1-6}$, halogène, cyano, trifluorométhyle, nitro, hydroxy ou alcoxy en $C_{1-6}$; et $R^{44}$ représente un groupe alkyle en $C_{1-6}$, halogène, cyano, trifluorométhyle, nitro, hydroxy ou alcoxy en $C_{1-6}$.

9. Composé selon la revendication 6 représenté par la formule IIC et les sels de ce composé:

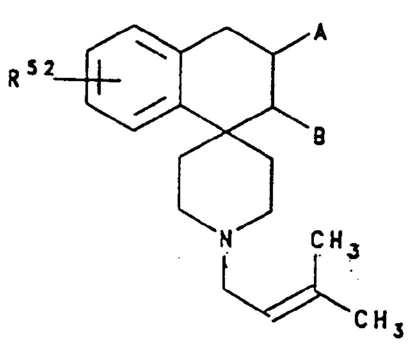

(IIC)

où A et B sont tels que définis dans la revendication 6; et $R^{52}$ représente l'hydrogène ou un groupe alkyle en $C_{1-6}$, halogène, cyano, trifluorométhyle, nitro, hydroxy ou alcoxy en $C_{1-6}$.

10. Composé selon la revendication 6 représenté par la formule IID et les sels de ce composé:

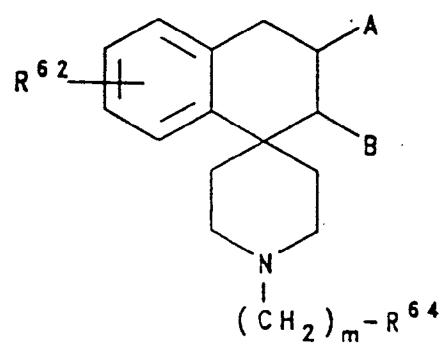

(I I D)

où A et B sont tels que définis dans la revendication 6; m est 1, 2 ou 3; $R^{64}$ est substitué en option par un groupe cycloalkyle en $C_{3-7}$; et $R^{62}$ représente l'hydrogène ou un groupe alkyle en $C_{1-6}$, halogène, cyano, trifluorométhyle, nitro, hydroxy ou alcoxy en $C_{1-6}$.

**11.** Composé selon la revendication 6 représenté par la formule IIE et les sels de ce composé:

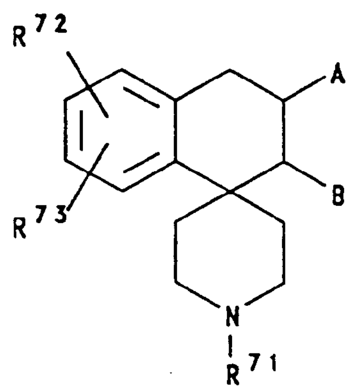

(I I E)

où A et B sont tels que définis dans la revendication 6; $R^{71}$ représente un groupe alcényle en $C_{4-6}$, hétérocycloalkyl($C_{3-7}$)alkyle en $C_{1-6}$ ou hétéroarylalkyle en $C_{1-6}$, l'un quelconque de ces groupes pouvant éventuellement être substitué; et $R^{72}$ et $R^{73}$ représentent indépendamment l'un de l'autre l'hydrogène ou un groupe alkyle en $C_{1-6}$, halogène, cyano, trifluorométhyle, nitro, hydroxy ou alcoxy en $C_{1-6}$.

**12.** Composé selon la revendication 6 choisi parmi les suivants:
1'-(2"-picolyl)spiro[1,2,3,4-tétrahydronaphtalène-1,4'-pipéridine];
1'-(4"-méthoxybenzyl)spiro[1,2,3,4-tétrahydronaphtalène-1,4'-pipéridine];
1'-(4"-méthylbenzyl)spiro[1,2,3,4-tétrahydronaphtalène-1,4'-pipéridine];
1'-benzylspiro[1,4-dihydro-7-méthoxynaphtalène-1,4'-pipéridine];
1'-cyclohexylméthylspiro[1,2,3,4-tétrahydronaphtalène- 1,4'-pipéridine];
1'-(4"-nitrobenzyl)spiro[1,2,3,4-tétrahydronaphtalène-1,4'-pipéridine];
1'-(4"-chlorobenzyl)spiro[1,2,3,4-tétrahydronaphtalène-1,4'-pipéridine];
1'-(2"-tétrahydrofurylméthyl)spiro[1,2,3,4-tétrahydronaphtalène-1,4'-pipéridine];
1'-but-3"énylspiro[1,2,3,4-tétrahydronaphtalène-1,4'-pipéridine];
1'-cyclopropylméthylspiro[1,2,3,4-tétrahydronaphtalène-1,4'-pipéridine];
1'-(3"méthylbut-2"ényl)spiro[1,2,3,4-tétrahydro- naphtalène-1,4'-pipéridine];
1'-(2"-furylméthyl)spiro[1,2,3,4-tétrahydronaphtalène-1,4'-pipéridine];
1'-(2"-thiénylméthyl)spiro[1,2,3,4-tétrahydronaphtalène-1,4'-pipéridine];
1'-benzylspiro[6-chloro-1,2,3,4-tétrahydronaphtalène-1,4'- pipéridine];
1'-benzylspiro[6-méthyl-1,2,3,4-tétrahydronaphtalène-1,4'-pipéridine];

1-benzylspiro[7-hydroxy-1,2,3,4-tétrahydronaphtalène- 1,4'-pipéridine];
et leurs sels.

**13.** Composé choisi parmi les suivants:
1'-n-butyl)spiro[1,2,3,4-tétrahydronaphtalène-1,4'-pipéridine];
1'-benzylspiro[7-méthoxy-1,2,3,4-tétrahydronaphtalène-1,4'-pipéridine;
1'-(2"-phényléthyl)spiro[1,2,3,4-tétrahydronaphtalène-1,4'-pipéridine];
1'-n-hexylspiro[1,2,3,4-tétrahydronaphtalène-1,4'-pipéridine];
1'-prop-2"-énylspiro[1,2,3,4-tétrahydronaphtalène-1,4'- pipéridine];
et leurs sels.

**14.** Composition pharmaceutique comprenant un composé selon la revendication 13 ou un sel pharmaceutiquement acceptable de celui-ci en association avec un ou plusieurs supports et/ou excipients pharmaceutiquement acceptables.

**15.** Composé selon la revendication 13 ou sel pharmaceutiquement acceptable de celui-ci pour utilisation en thérapie.

**16.** Procédé pour la préparation d'un composé de formule I tel que défini dans la revendication 1, lequel procédé comprend:

(A) pour la préparation d'un composé de formule I où A et B représentent chacun l'hydrogène:
la réduction d'un composé de formule III:

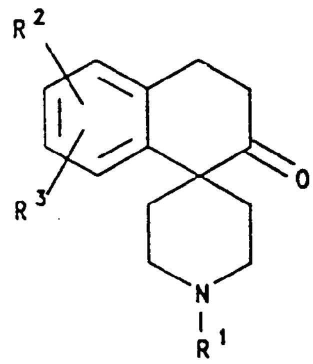

(III)

où $R^1$, $R^2$ et $R^3$ sont tels que définis dans la revendication 1; ou

(B) pour la préparation d'un composé de formule I où A et B représentent ensemble une liaison chimique:
la déshydratation d'un composé de formule V:

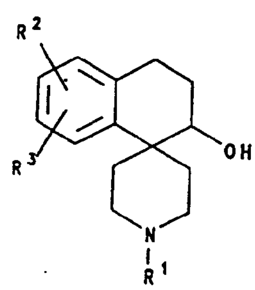

(V)

où $R^1$, $R^2$ et $R^3$ sont tels que définis dans la revendication 1; ou

(C) la réaction d'un composé de formule $R^1$-L avec un composé de formule VI:

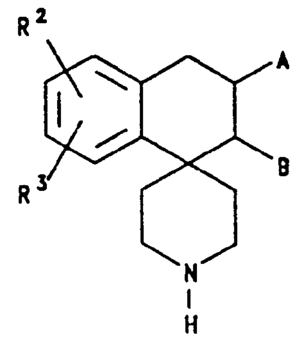

( V I )

où A, B, $R^1$, $R^2$ et $R^3$ sont tels que définis dans la revendication 1; et L représente un groupe séparable.

**Revendications pour les Etats contractants suivants : ES, GR**

1. Procédé pour la préparation d'un composé de formule I ou d'un sel pharmaceutiquement acceptable de celui-ci:

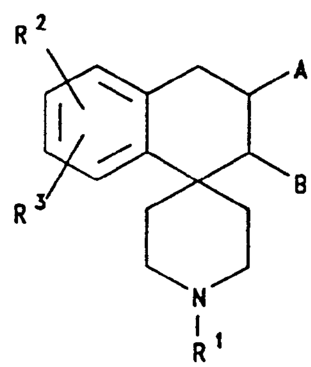

( I )

où

A et B représentent chacun l'hydrogène, ou A et B représentent ensemble une liaison chimique;

$R^1$ représente un groupe hydrocarboné;

$R^2$ et $R^3$ représentent, indépendamment l'un de l'autre, l'hydrogène ou un groupe hydrocarboné, halogène, cyano, trifluorométhyle, nitro, $-OR^x$, $-SR^x$, $-NR^xR^y$, $-CO_2R^x$ ou $-CONR^xR^y$, ou représentent ensemble un groupe méthylènedioxy; et

$R^x$ et $R^y$ représentent, indépendamment l'un de l'autre, l'hydrogène ou un groupe hydrocarboné;

le terme "hydrocarboné" tel qu'il est présentement utilisé comprenant des groupes à chaîne droite, ramifiés et cycliques, y compris des groupes hétérocycliques, contenant jusqu'à 18 atomes de carbone; lequel procédé comprend:

(A) pour la préparation d'un composé de formule I où A et B représentent chacun l'hydrogène:

la réduction d'un composé de formule III:

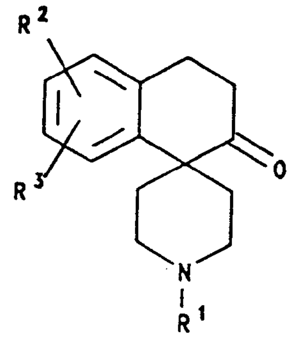

( I I I )

où $R^1$, $R^2$ et $R^3$ sont tels que définis plus haut; ou

(B) pour la préparation d'un composé de formule I où A et B représentent ensemble une liaison chimique:

la déshydratation d'un composé de formule V:

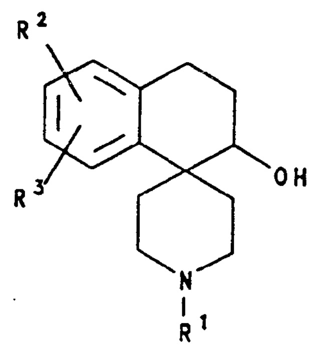

( V )

où $R^1$, $R^2$ et $R^3$ sont tels que définis plus haut; ou

(C) la réaction d'un composé de formule $R^1$-L avec un composé de formule VI:

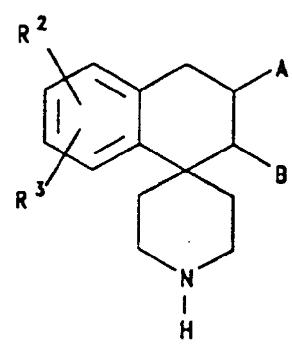

( V I )

où A, B, $R^1$, $R^2$ et $R^3$ sont tels que définis plus haut; et L représente un groupe séparable.

2. Utilisation d'un composé de formule I préparé comme décrit dans la revendication 1, ou d'un sel pharmaceutiquement acceptable de celui-ci, pour la préparation d'un médicament pour le traitement et/ou la prévention des troubles psychiatriques.

3. Utilisation selon la revendication 2, dans laquelle le composé de formule I est choisi parmi: la 1'-benzylspiro[1,2,3,4-tétrahydronaphtalène-1,4'-pipéridine] et des sels pharmaceutiquement acceptables de celle-ci.

4. Procédé pour la préparation d'une composition pharmaceutique, comprenant le mélange d'un composé de formule IA ou d'un sel pharmaceutiquement acceptable de ce composé:

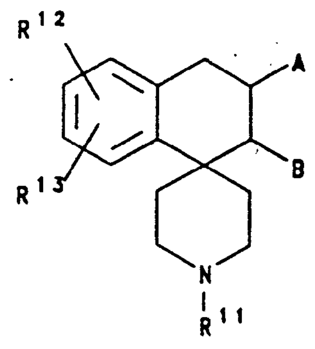

( I A )

où

A et B représentent chacun l'hydrogène, ou A et B représentent ensemble une liaison chimique;

$R^{11}$ représente un groupe hydrocarboné;

$R^{12}$ et $R^{13}$ représentent, indépendamment l'un de l'autre, l'hydrogène ou un groupe hydrocarboné, halogène, cyano, trifluorométhyle, nitro, $-OR^x$, $-SR^x$, $-NR^xR^y$, $-CO_2R^x$ ou $-CONR^xR^y$, ou représentent ensemble un groupe méthylènedioxy; et

$R^x$ et $R^y$ représentent, indépendamment l'un de l'autre, l'hydrogène ou un groupe hydrocarboné;

à la condition que, lorsque A et B représentent tous les deux l'hydrogène et que $R^{11}$ représente un groupe alkyle, allyle, aryle, aralkyle, cycloalkyle ou ar(cycloalkyle), alors $R^{12}$ et $R^{13}$ ne représentent pas l'hydrogène ou un groupe alcoxy ou hydroxyalkyle;

à la condition également que, lorsque A, B et $R^{12}$ représentent chacun l'hydrogène et que $R^{11}$ est un groupe de formule $-CH_2CH_2CX^1Ph_2$ dans laquelle $X^1$ représente un groupe cyano ou alkyl$(C_{2-7})$carbonyle, alors $R^{13}$ ne représente pas l'hydrogène ou un groupe méthyle sur la position 7 de la partie tétrahydronaphtalène;

à la condition en outre que, lorsque $R^{11}$ est un groupe de formule $-CH_2CH_2CX^2Ph_2$ dans laquelle $X^2$ représen- te un groupe diméthylaminocarbonyle, 1-hydroxypropyle ou 1-acétoxypropyle, alors A, B, $R^{12}$ et $R^{13}$ ne représentent pas simultanément l'hydrogène;

le terme "hydrocarboné" tel qu'il est présentement utilisé comprenant des groupes à chaîne droite, ramifiés et cycliques, y compris des groupes hétérocycliques, contenant jusqu'à 18 atomes de carbone; avec un ou plusieurs supports et/ou excipients pharmaceutiquement acceptables.

5. Procédé selon la revendication 4 pour la préparation d'une composition pharmaceutique, où le constituant actif est la 1'-benzylspiro[1,4-dihydro- naphtalène-1,4'-pipéridine] ou un sel pharmaceutiquement acceptable de celle-ci.

6. Procédé selon la revendication 1 pour la préparation d'un composé de formule II ou d'un sel de ce composé:

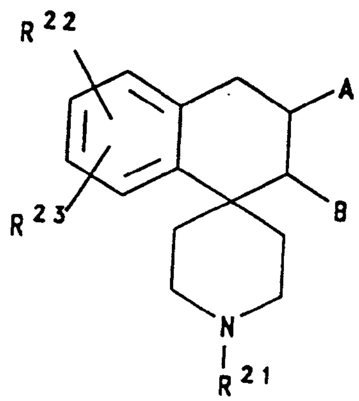

(II)

où

A et B représentent chacun l'hydrogène, ou A et B représentent ensemble une liaison chimique;

$R^{21}$ représente un groupe hydrocarboné;

le terme "hydrocarboné" tel qu'il est présentement utilisé comprenant des groupes à chaîne droite, ramifiés ou cycliques, y compris des groupes hétérocycliques, contenant jusqu'à 18 atomes de carbone.

$R^{22}$ et $R^{23}$ représentent, indépendamment l'un de l'autre, l'hydrogène ou un groupe hydrocarboné, halogène, cyano, trifluorométhyle, nitro, $-OR^x$, $-SR^x$, $-NR^xR^y$, $-CO_2R^x$ ou $-CONR^xR^y$, ou représentent ensemble un groupe méthylènedioxy; et

$R^x$ et $R^y$ représentent, indépendamment l'un de l'autre, l'hydrogène ou un groupe hydrocarboné;

à condition que, lorsque A et B représentent tous les deux l'hydrogène et que $R^{21}$ représente un groupe alkyle, allyle, aryle, aralkyle, cycloalkyle ou ar(cycloalkyle), alors $R^{22}$ et $R^{23}$ ne représentent pas l'hydrogène ou un groupe alcoxy ou hydroxyalkyle;

à la condition également que, lorsque A, B et $R^{22}$ représentent chacun l'hydrogène et que $R^{21}$ est un groupe méthyle, alors $R^{23}$ ne représente pas un groupe hydroxy sur la position 6 de la partie tétrahydronaphtalène;

à la condition également que, lorsque A, B et $R^{22}$ représentent chacun l'hydrogène et que $R^{21}$ est un groupe benzyle, alors $R^{23}$ ne représente pas un groupe méthyle sur la position 7 de la partie tétrahydronaphtalène;

à la condition également que, lorsque A, B et $R^{22}$ représentent chacun l'hydrogène et que $R^{21}$ est un groupe de formule $-CH_2CH_2CX^1Ph_2$ dans laquelle $X^1$ représente un groupe cyano ou alkyl($C_{2-7}$)carbonyle, alors $R^{23}$ ne représente pas l'hydrogène ou un groupe méthyle sur la position 7 de la partie tétrahydronaphtalène;

à la condition également que, lorsque $R^{21}$ est un groupe de formule $-CH_2CH_2CX^2Ph_2$ dans laquelle $X^2$ représen- te un groupe diméthylaminocarbonyle, 1-hydroxypropyle ou 1-acétoxypropyle, alors A, B, $R^{22}$ et $R^{23}$ ne représentent pas simultanément l'hydrogène;

à la condition en outre que, lorsque A et B représentent ensemble une liaison chimique et que $R^{21}$ représente un groupe benzyle, alors $R^{22}$ et $R^{23}$ ne représentent pas simultanément l'hydrogène.

7. Procédé selon la revendication 6 pour la préparation d'un composé représenté par la formule IIA et de sels de ce composé:

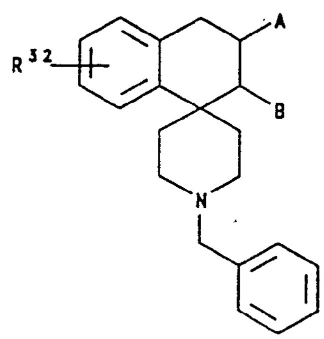

(IIA)

où A et B sont tels que définis dans la revendication 6; et $R^{32}$ représente un groupe alkyle en $C_{1-6}$, halogène, cyano, trifluorométhyle, nitro ou hydroxy.

8. Procédé selon la revendication 6 pour la préparation d'un composé représenté par la formule IIB et de sels de ce composé:

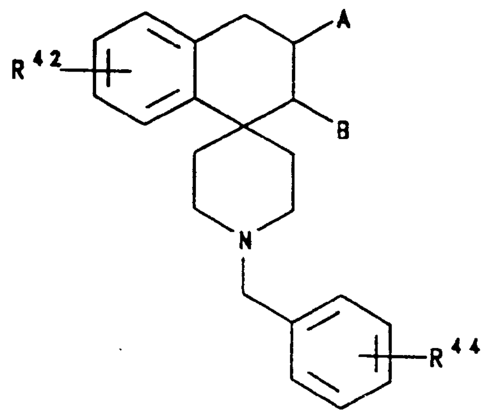

(IIB)

où A et B sont tels que définis dans la revendication 6; $R^{42}$ représente l'hydrogène ou un groupe alkyle en $C_{1-6}$ halogène, cyano, trifluorométhyle, nitro, hydroxy ou alcoxy en $C_{1-6}$; et $R^{44}$ représente un groupe alkyle en $C_{1-6}$, halogène, cyano, trifluorométhyle, nitro, hydroxy ou alcoxy en $C_{1-6}$.

9. Procédé selon la revendication 6 pour la préparation d'un composé représenté par la formule IIC et de sels de ce composé:

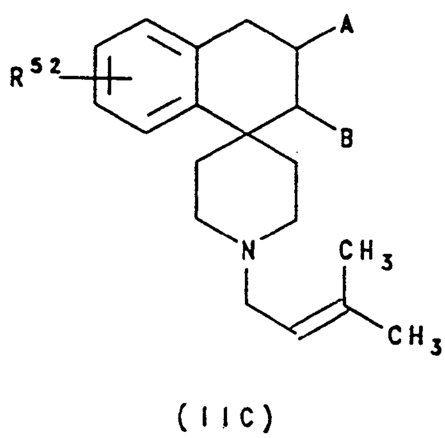

(IIC)

où A et B sont tels que définis dans la revendication 6; et $R^{52}$ représente l'hydrogène ou un groupe alkyle en $C_{1-6}$, halogène, cyano, trifluorométhyle, nitro, hydroxy ou alcoxy en $C_{1-6}$.

**10.** Procédé selon la revendication 6 pour la préparation d'un composé représenté par la formule IID et de sels de ce composé:

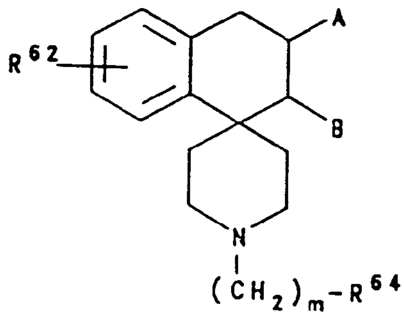

(IID)

où A et B sont tels que définis dans la revendication 6; m est 1, 2 ou 3; $R^{64}$ est substitué en option par un groupe cycloalkyle en $C_{3-7}$; et $R^{62}$ représente l'hydrogène ou un groupe alkyle en $C_{1-6}$, halogène, cyano, trifluorométhyle, nitro, hydroxy ou alcoxy en $C_{1-6}$.

**11.** Procédé selon la revendication 6 pour la préparation d'un composé représenté par la formule IIE et de sels de ce composé:

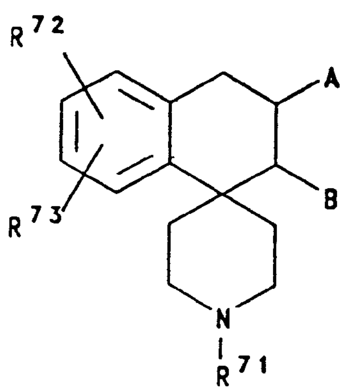

(IIE)

où A et B sont tels que définis dans la revendication 6; $R^{71}$ représente un groupe alcényle en $C_{4-6}$, hétérocycloalkyl($C_{3-7}$)alkyle en $C_{1-6}$ ou hétéroarylalkyle en $C_{1-6}$, l'un quelconque de ces groupes pouvant éventuellement être substitué; et $R^{72}$ et $R^{73}$ représentent indépendamment l'un de l'autre l'hydrogène ou un groupe alkyle en $C_{1-6}$, halogène, cyano, trifluorométhyle, nitro, hydroxy ou alcoxy en $C_{1-6}$.

**12.** Procédé selon la revendication 6 pour la préparation d'un composé choisi parmi les suivants:
1'-(2"-picolyl)spiro[1,2,3,4-tétrahydronaphtalène-1,4'-pipéridine];
1'-(4"-méthoxybenzyl)spiro[1,2,3,4-tétrahydronaphtalène-1,4'-pipéridine];
1'-(4"-méthylbenzyl)spiro[1,2,3,4-tétrahydronaphtalène-1,4-'pipéridine];
1'-benzylspiro[1,4-dihydro-7-méthoxynaphtalène-1,4'-pipéridine];
1'-cyclohexylméthylspiro[1,2,3,4-tétrahydronaphtalène-1,4'-pipéridine];
1'(-4"-nitrobenzyl)spiro[1,2,3,4-tétrahydronaphtalène-1,4'-pipéridine];
1'-(4"-chlorobenzyl)spiro[1,2,3,4-tétrahydronaphtalène-1,4'-pipéridine];
1'-(2"-tétrahydrofurylméthyl)spiro[1,2,3,4-tétrahydronaphtalène-1,4'-pipéridine];
1'-but-3"énylspiro[1,2,3,4-tétrahydronaphtalène-1,4'-pipéridine];

1'-cyclopropylméthylspiro[1,2,3,4-tétrahydronaphtalène-1,4'-pipéridine];
1'-(3"méthylbut-2"ényl)spiro[1,2,3,4-tétrahydronaphtalène-1,4'-pipéridine];
1'-(2"-furylméthyl)spiro[1,2,3,4-tétrahydronaphtalène-1,4'-pipéridine];
1'-(2"-thiénylméthyl)spiro[1,2,3,4-tétrahydronaphtalène-1,4'-pipéridine];
1'-benzylspiro[6-chloro-1,2,3,4-tétrahydronaphtalène-1,4'-pipéridine];
1'-benzylspiro[6-méthyl-1,2,3,4-tétrahydronaphtalène-1,4'-pipéridine];
1'-benzylspiro[7-hydroxy-1,2,3,4-tétrahydronaphtalène-1,4'pipéridine];
et leurs sels.

**13.** Procédé selon la revendication 1 pour la préparation d'un composé choisi parmi les suivants:
1'-(n-butyl)spiro[1,2,3,4-tétrahydronaphtalène-1,4'-pipéridine];
1'-benzylspiro[7-méthoxy-1,2,3,4-tétrahydronaphtalène-1,4'-pipéridine];
1'-(2"-phényléthyl)spiro[1,2,3,4-tétrahydronaphtalène-1,4'-pipéridine];
1'-n-hexylspiro[1,2,3,4-tétrahydronaphtalène-1,4'-pipéridine];
1'-prop-2"-énylspiro[1,2,3,4-tétrahydronaphtalène-1,4'-pipéridine];
et leurs sels.

**14.** Procédé pour la préparation d'une composition pharmaceutique, comprenant le mélange d'un composé préparé selon la revendication 13 ou d'un sel pharmaceutiquement acceptable de celui-ci avec un ou plusieurs supports et/ou excipients pharmaceutiquement acceptables.